# EUROPEAN PATENT SPECIFICATION

(11) **EP 4 087 879 B1**
(45) Date of publication and mention of the grant of the patent: **06.05.2026**
(21) Application number: 21717562.9
(22) Date of filing: 11.03.2021
(51) Int. Cl.: C07K 16/30, A61K 47/68

(54) **ANTI-MUCI-SEA ANTIBODIES**
ANTIKÖRPER GEGEN ANTI-MUCI-SEA
ANTI-MUCI-SEA ANTIBODIES

(30) Priority: 18.03.2020 US 202062991077 P
(43) Date of publication of application: 16.11.2022
(73) Proprietor: Biomodifying LLC, Silver Spring, Maryland 20902 (US); Ramot at Tel-Aviv University Ltd., Tel-Aviv 6139201 (IL)
(72) Inventor: RUBINSTEIN, Daniel, Silver Spring, Maryland 20902 (US); WRESCHNER, Daniel, 9043500 Efrat (IL)
(74) Representative: Symbiosis IP Limited
(86) International application number: PCT/IL2021/050269
(87) International publication number: WO 2021/186427

(56) References cited:
- EP-A2- 3 604 334
- WO-A2-2006/081553
- RUBINSTEIN DANIEL B ET AL: "MUC1/X protein immunization enhances cDNA immunization in generating anti-MUC1 alpha/beta junction antibodies that target malignant cells", CANCER RESEARCH, AMERICAN ASSOCIATION FOR CANCER RESEARCH, US, vol. 66, no. 23, 1 December 2006 (2006-12-01), pages 11247 - 11253, XP002522189, ISSN: 0008-5472, DOI: 10.1158/0008-5472.CAN-06-1486
- E. PICHINUK ET AL: "Antibody Targeting of Cell-Bound MUC1 SEA Domain Kills Tumor Cells", CANCER RESEARCH, vol. 72, no. 13, 1 July 2012 (2012-07-01), pages 3324 - 3336, XP055045742, ISSN: 0008-5472, DOI: 10.1158/0008-5472.CAN-12-0067
- RUBINSTEIN DANIEL B ET AL: "The MUC1 oncoprotein as a functional target: immunotoxin binding to alpha/beta junction mediates cell killing", INTERNATIONAL JOURNAL OF CANCER, MALDEN, MASS : WILEY-BLACKWELL, US, vol. 124, no. 1, 1 January 2009 (2009-01-01), pages 46 - 54, XP002769039, ISSN: 1097-0215, DOI: 10.1002/IJC.23910
- PICHINUK EDWARD ET AL: "In vivo anti-MUC1tumor activity and sequences of high-affinity anti-MUC1-SEA antibodies", CANCER IMMUNOLOGY, IMMUNOTHERAPY, SPRINGER, BERLIN/HEIDELBERG, vol. 69, no. 7, 26 March 2020 (2020-03-26), pages 1337 - 1352, XP037168525, ISSN: 0340-7004, [retrieved on 20200326], DOI: 10.1007/S00262-020-02547-2

## Description

### TECHNOLOGICAL FIELD

The present invention relates to antibodies specifically directed against the junction of the alpha and beta chains (which comprises the SEA domain) of MUC1, a glycoprotein present on the cell surface of human cells and to methods and uses thereof in the detection and treatment of cancer, as well as in the detection and treatment of a variety of non-malignant diseases and disorders.

### BACKGROUND ART

References considered to be relevant as background to the presently disclosed subject matter are listed below:
[1] G. Rivalland, B. Loveland, P. Mitchell, Update on Mucin-1 immunotherapy in cancer: a clinical perspective, Expert Opin Biol Ther, 15 (2015) 1773-1787.
[2] J. Taylor-Papadimitriou, J.M. Burchell, R. Graham, R. Beatson, Latest developments in MUC1 immunotherapy, Biochem Soc Trans, 46 (2018) 659-668.
[3] J.M. Burchell, A. Mungul, J. Taylor-Papadimitriou, O-linked glycosylation in the mammary gland: changes that occur during malignancy, J Mammary Gland Biol Neoplasia, 6 (2001) 355-364.
[4] W. Fiedler, S. DeDosso, S. Cresta, J. Weidmann, A. Tessari, M. Salzberg, B. Dietrich, H. Baumeister, S. Goletz, L. Gianni, C. Sessa, A phase I study of PankoMab-GEX, a humanised glyco-optimised monoclonal antibody to a novel tumour-specific MUC1 glycopeptide epitope in patients with advanced carcinomas, Eur J Cancer, 63 (2016) 55-63.
[5] K. Ryuko, D.J. Schol, F.G. Snijdewint, S. von Mensdorff-Pouilly, R.J. Poort-Keesom, Y.A. Karuntu-Wanamarta, R.A. Verstraeten, K. Miyazaki, P. Kenemans, J. Hilgers, Characterization of a new MUC1 monoclonal antibody (VU-2-G7) directed to the glycosylated PDTR sequence of MUC1, Tumour Biol, 21 (2000) 197-210.
[6] M.A. Tarp, A.L. Sorensen, U. Mandel, H. Paulsen, J. Burchell, J. Taylor-Papadimitriou, H. Clausen, Identification of a novel cancer-specific immunodominant glycopeptide epitope in the MUC1 tandem repeat, Glycobiology, 17 (2007) 197-209.
[7] D. Zhou, L. Xu, W. Huang, T. Tonn, Epitopes of MUC1 Tandem Repeats in Cancer as Revealed by Antibody Crystallography: Toward Glycopeptide Signature-Guided Therapy, Molecules, 23 (6) (2018) 1326.
[8] T. Kimura, O.J. Finn, MUC1 immunotherapy is here to stay, Expert Opin Biol Ther, 13 (2013) 35-49.
[9] N.K. Ibrahim, K.O. Yariz, I. Bondarenko, A. Manikhas, V. Semiglazov, A. Alyasova, V. Komisarenko, Y. Shparyk, J.L. Murray, D. Jones, S. Senderovich, A. Chau, F. Erlandsson, G. Acton, M. Pegram, Randomized phase II trial of letrozole plus anti-MUC1 antibody AS1402 in hormone receptor-positive locally advanced or metastatic breast cancer, Clin Cancer Res, 17 (2011) 6822-6830.
[10] D.B. Rubinstein, M. Karmely, R. Ziv, I. Benhar, O. Leitner, S. Baron, B.Z. Katz, D.H. Wreschner, MUC1/X protein immunization enhances cDNA immunization in generating anti-MUC1 alpha/beta junction antibodies that target malignant cells, Cancer Res, 66 (2006) 11247-11253.
[11] E. Pichinuk, I. Benhar, O. Jacobi, M. Chalik, L. Weiss, R. Ziv, C. Sympson, A. Karwa, N.I. Smorodinsky, D.B. Rubinstein, D.H. Wreschner, Antibody targeting of cell-bound MUC1 SEA domain kills tumor cells, Cancer Res, 72 (2012) 3324-3336.
[12] D.B. Rubinstein, M. Karmely, E. Pichinuk, R. Ziv, I. Benhar, N. Feng, N.I. Smorodinsky, D.H. Wreschner, The MUC1 oncoprotein as a functional target: immunotoxin binding to alpha/beta junction mediates cell killing, Int J Cancer, 124 (2009) 46-54.
[13] D.V. Gold, Z. Karanjawala, D.E. Modrak, D.M. Goldenberg, R.H. Hruban, PAM4-reactive MUC1 is a biomarker for early pancreatic adenocarcinoma, Clin Cancer Res, 13 (2007) 7380-7387.

Acknowledgement of the above references herein is not to be inferred as meaning that these are in any way relevant to the patentability of the presently disclosed subject matter.

### BACKGROUND

The MUC1 glycoprotein is over-expressed by a variety of high-incidence, high-mortality human epithelial malignancies, including breast, prostate, pancreas, ovarian, lung, and colon carcinomas, as well as by the malignant plasma cells of multiple myeloma, and by the myelogenous cells of acute myelogenous leukemia. Because of its preferentially high expression by malignant cells, and because it is expressed on the cell surface and therefore is an exposed molecule, MUC1 has been studied as both a target for directed cancer therapy and as a marker of disease progression [1, 2].

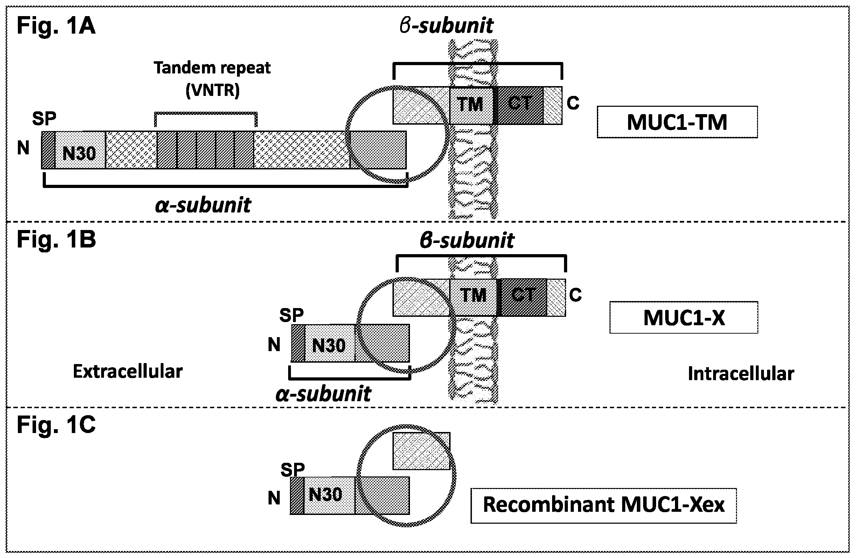

Structurally, the MUC1 molecule is a transmembrane glycoprotein (termed MUC-TM). MUC-TM is a heterodimer consisting of an extracellular domain containing between 20 to 125 repeats of a 20 amino acid-long sequence (termed the variable number tandem repeat, VNTR), a transmembrane domain, and a short cytoplasmic tail mediating intracellular signaling (see Figure 1A). The MUC1 molecule is auto-proteolytically cleaved within the SEA module, a highly-conserved domain of 120 amino acids. This results in a large extracellular α subunit containing the tandem repeat array bound in a strong non-covalent interaction to a transmembrane β subunit containing the transmembrane and cytoplasmic domains of the molecule. Binding of the α chain to the β chain is intermittent: The α chain binds the β chain in an on-and-off manner. While the β chain remains on the cell surface all the time, the α chain with its VNTR remains cell-bound only intermittently.

A number of anti-MUC1 antibodies have been reported in the literature with the great majority directed against the highly immunogenic VNTR of the α chain. While anti-VNTR antibodies can successfully bind MUC1+ cells *in vitro,* the shedding of the MUC1 α chain containing the VNTR into the peripheral circulation *in vivo* severely compromises the ability of anti-VNTR antibodies to clinically affect MUC1 expressing tumors. Shedding of the α chain off the tumor cell surface not only greatly decreases the number of MUC1 targets for anti-α chain antibodies, but additionally, freely circulating MUC1 α chain *in vivo* in the periphery can bind and neutralize anti-VNTR antibodies or anti-glycosylated-VNTR antibodies thereby limiting their ability to even reach MUC1-expressing tumor. Antibodies that recognize cancer-specific truncated O-glycoforms of the VNTR, such as the antibodies PankoMab-Gex, 5E5, SM3, and VU-2-G7, have been proposed as possible ways to overcome the potential toxicity of targeting MUC1 expressed by normal tissues. However, the limitations of targeting the α-chain VNTR (Fig 1A), namely its shedding from the cell surface and its ability to bind therapeutically administered anti-MUC1 antibody, remain [3-7]. Further anti-MUC1 antibodies are known in the art. For example, WO2006/081553 discloses antibodies that bind to MUC1-SEA to detect membrane bound MUC1. WO2006/081553 does not disclose the sequence of the MUC1-SEA antibodies. The disclosure in WO2006/081553 is published in Rubinstein et al (2006) Cancer Research, American Association for Cancer Research, US VOL 66(23) pp11247 to 11253. Pichinuk et al (2012) Cancer Research vol 72(13) pp3324 to 3336 discloses MUC1 antibodies that bind to the MUC1-SEA domain of MUC1 although there is no disclosure of the sequence of these antibodies. EP3604334A2 discloses MUC1 antibodies which bind MUC1 but does not disclose antibodies that bind to MUC1-SEA domain. Rubinstein et al (2009), International Journal of Cancer, US, vol124(1), pp46 to 54 discloses MUC1-SEA antibodies and cytotoxic activity against MUC1 expressing cells but does not disclose the antibodies designated DMB5F3, DMB7F3, DMB4B4, DMB10F10, DMB4F4, DMB10B7 and DMBD11 in the present disclosure.

Because of the instability of their target, which, as noted, binds the tumor cell only intermittently in an on-and off mechanism, no anti-MUC1 VNTR antibody has yet been proven to be clinically effective [4, 8, 9]. Of note, Fiedler et al reported 16 cases of stable disease in a clinical trial of anti-VNTR cancer-specific glycosylation antibody PankoMab-Gex [4]. However, that study was a Phase I trial of the anti-VNTR antibody, where the primary study objective was antibody safety rather than anti-tumor efficacy, such that the cases of stable disease observed cannot be truly interpreted. To date, no antibodies against the MUC1 VNTR, nor any antibodies against the α chain of MUC1 has ever been shown to be effective against tumors in humans.

In contrast to the α chain and its VNTR, the MUC1 SEA domain formed by the interaction of the α-subunit with the extracellular portion of the β-subunit is a stable cell membrane-fixed molecular moiety. Anti-MUC1 alpha/beta junction antibodies were disclosed in Rubinstein et al and in Pichinuk et al [10-12].

### GENERAL DESCRIPTION

In a first aspect, the present invention provides an isolated monoclonal antibody or antigen-binding fragment thereof which binds to the MUC1 SEA domain, wherein said antibody comprises:
a heavy chain complementarity determining region (CDRH) 1 denoted by SEQ ID NO. 25, CDRH2 denoted by SEQ ID NO. 26, CDRH3 denoted by SEQ ID NO. 27, and a light chain complementarity determining region (CDRL) 1 denoted by SEQ ID NO. 28, a CDRL2 denoted by SEQ ID NO. 29, and a CDRL3 denoted by SEQ ID NO. 30.

In some embodiments, said antibody comprises a heavy chain variable region and a light chain variable region, wherein:
said heavy chain variable region is encoded by a nucleic acid sequence which is at least 70% identical to the nucleic acid sequence denoted by SEQ ID NO. 1 and wherein said light chain variable region is encoded by a nucleic acid sequence which is at least 70, 80 or 90% identical to SEQ ID NO. 2.

In some embodiments, said antibody comprises:
a heavy chain variable region comprising the amino acid sequence denoted by SEQ ID NO. 13 or a variant thereof and a light chain variable region comprising the amino acid sequence denoted by SEQ ID NO. 14, or a variant thereof

In some embodiments, said antibody is a complete mouse antibody, a complete chimeric antibody, or a complete humanized antibody (e.g., wherein parts of the antibody sequence are derived from mouse sequence and part from human).

In some embodiments, said antigen-binding fragment thereof is Fv, single chain Fv (scFv), single chain Fv-Fc (scFv-Fc), Fab', Fab, F(ab')₂ or F(ab)₂.

In some embodiments, said antibody or antigen-binding fragment thereof identify MUC1 SEA in immunohistochemistry performed on formaldehyde fixed sections from Fresh Frozen (FF) tissues, and/or on Paraffin Embedded and Formaldehyde Fixed (PEFF) tissues, and/or by fluorescence-activated cell sorting (FACS) analysis of MUC1-expressing human cells.

In another embodiment, the present invention provides an isolated nucleic acid molecule comprising a nucleotide sequence encoding the antibody of the invention as described herein or any antigen-binding fragment thereof.

In another embodiment, the present invention provides an expression vector comprising the isolated nucleic acid molecule of the invention.

In another embodiment, the present invention provides a host cell transfected with the expression vector of the invention.

In another embodiment, the present invention provides an immunoconjugate comprising the antibody of the invention or antigen-binding fragment thereof and an additional cytotoxic or therapeutic agent.

In certain embodiments, said cytotoxic agent is selected from a group consisting of alkylating drugs, anthracyclines, pyrimidine derivatives, vinca alkaloids, photodynamic drugs, platinum-containing compounds, taxanes, topoisomerase inhibitors, ribosome inactivating agents, agents that induce DNA damage, tubulin inhibitors, anti-mitotic agents, radioisotopes, cytotoxic antibodies, and bacterial toxins.

In one embodiment, said cytotoxic agent is pseudomonas exotoxin.

In one embodiment, said immunoconjugate reduces tumor volume upon administration to a subject with cancer.

In another embodiment, the present invention provides a bispecific antibody comprising the antibody of the invention or antigen-binding fragment thereof bound to a second antibody binding a different antigen target.

In another embodiment, the present invention provides a pharmaceutical composition comprising as an active ingredient the isolated monoclonal antibody or antigen-binding fragment thereof of the invention, or the immunoconjugate of the invention, or the bispecific antibody of the invention and a pharmaceutically acceptable carrier, excipient, or diluent.

In an embodiment, said pharmaceutical composition is for use in the treatment of cancer.

In an embodiment, said pharmaceutical composition further comprises an additional therapeutic agent.

In some embodiments, the present invention provides, a method of treatment or amelioration of a disease or disorder comprising administering to a subject in need thereof a therapeutically effective amount of at least one isolated monoclonal antibody or antigen-binding fragment thereof, or the immunoconjugate, or the bispecific antibody, or the pharmaceutical composition of the invention.

In an embodiment, said method further comprises administering to a subject in need thereof an additional therapeutic agent.

In one embodiment, said cancer is a MUC1 expressing cancer.

In some embodiments, said cancer is selected from the group consisting of lung carcinoma, prostate carcinoma, breast carcinoma, ovarian carcinoma, colon carcinoma, pancreatic carcinoma, multiple myeloma, and acute myelogenous leukemia.

All the recited cancer types are known to express MUC1 on their malignant cells.

In some embodiments, the present invention provides the isolated monoclonal antibody or antigen-binding fragment thereof, or the immunoconjugate, or the bispecific antibody, or the pharmaceutical composition of the invention for use in a method of treatment or amelioration of a disease or disorder, said method comprising administering to a subject in need thereof a therapeutically effective amount of said isolated monoclonal antibody or antigen-binding fragment thereof, said immunoconjugate, said bispecific antibody, or said pharmaceutical composition.

In one embodiment, said method further comprises administering to a subject in need thereof an additional therapeutic agent.

In one embodiment said disease or disorder is a MUC1 expressing cancer.

In another aspect, the present invention provides a method of diagnosing a disease or disorder in a subject, wherein said disease or disorder is associated with MUC-1 expression, said method comprising:
a. contacting a biopsy obtained from said patient with at least one isolated monoclonal antibody or antigen-binding fragment thereof of the invention; and
b. detecting said isolated monoclonal antibody or any antigen-binding fragment thereof;
wherein detection of cells over-expressing MUC1 SEA in the biopsy indicates that the subject is diagnosed with a disease or disorder.

In one embodiment said disease or disorder is cancer.

In one embodiment, said isolated monoclonal antibody or antigen-binding fragment thereof is detectably labelled.

According to a further aspect of the invention there is provided an isolated anti-MUC1 SEA monoclonal antibody or antigen-binding fragment thereof, for use as an imaging agent to image a disease or disorder associated with MUC1 expression in a subject in need thereof, wherein said antibody or antigen-binding fragment thereof are detectably labelled with a radioisotope or a visualizable agent in order to be visualized, and wherein the detection of cells and/or tissues labelled with said isotope or visualizable agent indicates the presence, the location and/or extent of metastases of said disease or disorder in said subject.

In a preferred embodiment of the invention said disease is a MUC1 expressing cancer.

### BRIEF DESCRIPTION OF THE DRAWINGS

To better understand the subject matter that is disclosed herein and to exemplify how it may be carried out in practice, embodiments will now be described by way of non-limiting example only, with reference to the accompanying drawings:
**Figures 1A-1C** are schematic representations of the MUC1-TM, the MUC1-X isoform, and recombinant MUC1-X molecules. (1A) Going from the N (N) to the C terminus (C), MUC1-TM is composed of an N-terminal Signal Peptide, followed by a 30 amino acid-long segment (N30), leading into sequences that N-terminally and C-terminally flank the variable tandem repeat array (VNTR). This is followed by a region common to both the MUC1-X isoform (1B) and to the soluble extracellular domain of MUC1 X, MUC1-Xex (1C). The β-subunit extracellular domain consists of 58 amino acids immediately N-terminal to the transmembrane (TM) and cytoplasmic (CT) domains. The SEA module comprises 120 amino acids, contributed by both the α- and β-subunits. Recombinant soluble MUC1-Xex protein (1C) includes the signal peptide, the N-terminal 30 amino acid sequence and the SEA module regions.

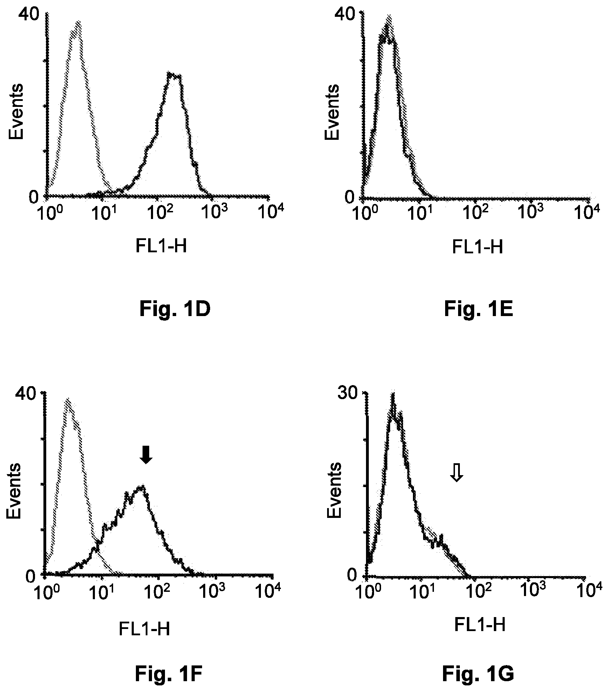

**Figures 1D-1K** are graphs showing flow cytometric analyses of the anti-MUC1 SEA antibody DMB5F3. DA3 cells stably transfected with MUC1-TM (1D) or non-transfected DA3 cells (1E) were reacted with anti-MUC1 SEA mAb DMB5F3, followed by an FITC-conjugate (tracings marked by arrow). MUC1+ human pancreatic cancer cell line Colo357 (1F) and MUC1+ breast cancer cells T47D and ZR75 (1H, 1J) were reacted with DMB5F3 (tracings marked by arrow). In all four panels, the tracings not marked by arrow represent cells bound to secondary antibody alone, and the tracings marked by arrow that of cells reacted with both DMB5F3 and secondary antibody. Anti-MUC1 SEA binding to MUC1+ cells was entirely competed-out by the presence of soluble MUC1-Xex protein (MUC1-Xex-mediated abrogated binding is indicated by empty arrows in 1G, 1I, and 1K).

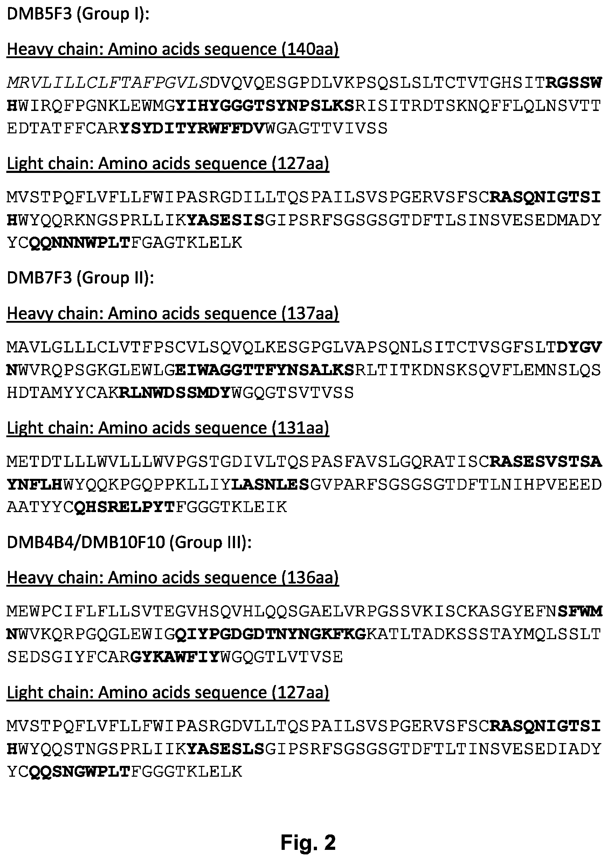

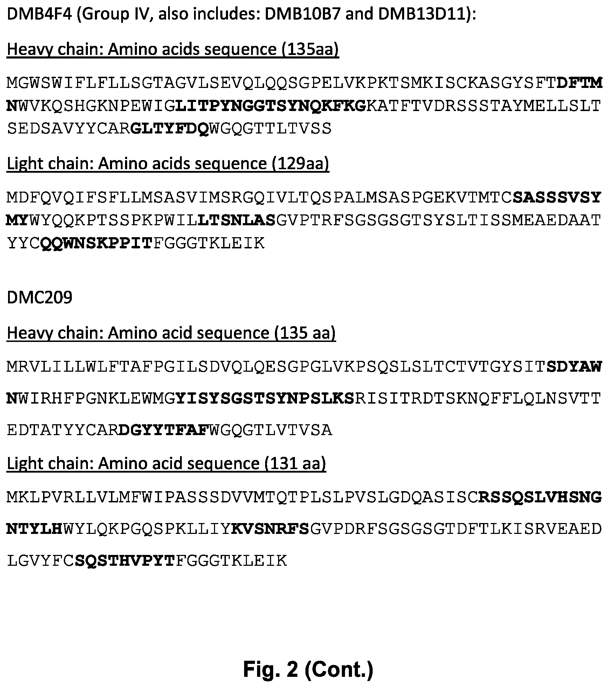

**Figure 2** shows the amino acid sequences of the variable regions of the anti-MUC1 SEA α-β junction monoclonal antibodies. For all sequences, the following general structure is presented: *Leader sequence*-FR1-**CDR1**-FR2-**CDR2**-FR3-**CDR3**-FR4.

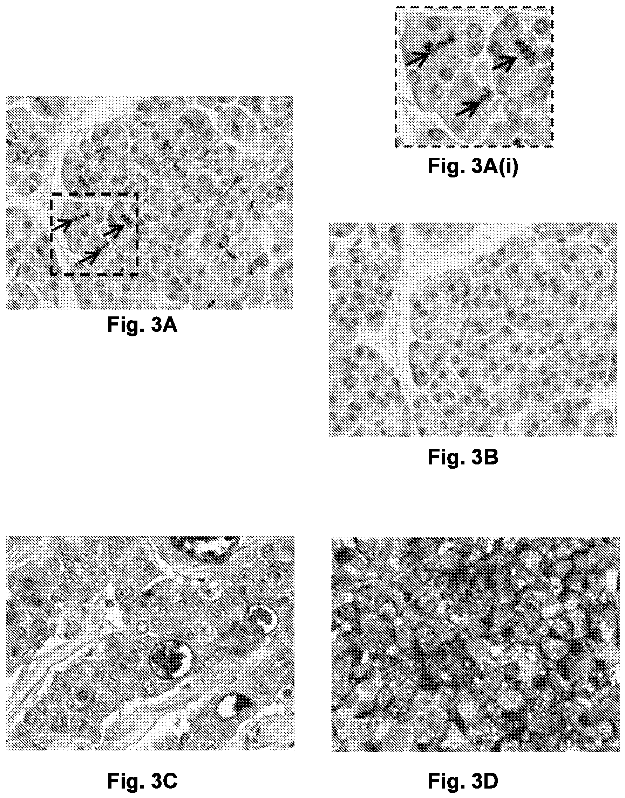

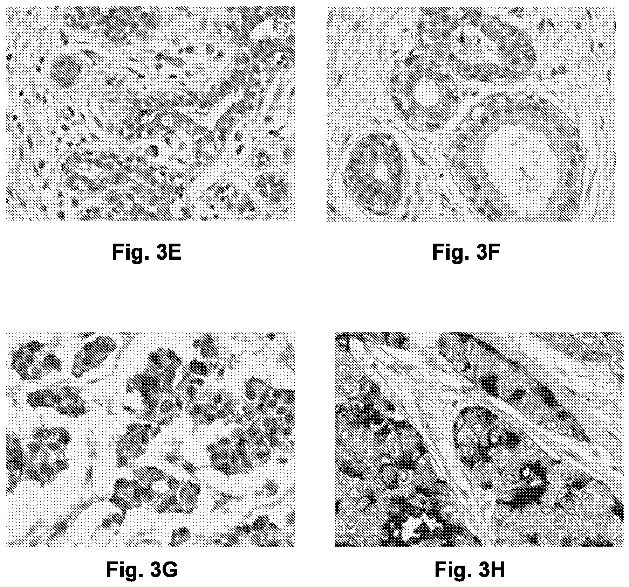

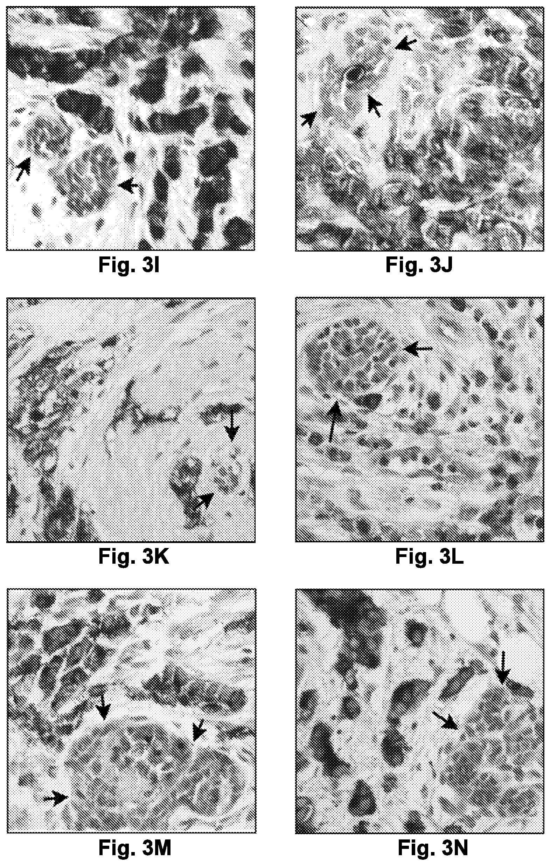

**Figures 3A-3N** show the architecture of MUC1 expression delineated by the anti-MUC1 SEA α-β junction antibody DMB5F3. **Figures 3A-3D****:** Paraffin-embedded microarrays of normal (3A and 3B) and malignant pancreatic tissue (3C and 3D) were stained with DMB5F3. DMB5F3 strongly stained malignant cells in a near-circumferential pattern (3C and 3D), while in normal pancreatic acinar cells only weak apical positivity was seen (3A(i), black arrows). Addition of MUC1-Xex protein together with DMB5F3 competitively abrogated staining (3B), indicating the specificity of DMB5F3 binding. **Figures 3E-3H****:** Paraffin-embedded normal breast tissues (3E and 3F) and malignant invasive ductal breast adenocarcinomas (3G and 3H) from 4 patients were stained with the DMB5F3 antibody. **Figures 3I-3N** Breast carcinoma biopsy specimens of 6 patients, each consisting of tumor surrounded by adjacent non-malignant tissue, stained with DMB5F3. In each specimen DMB5F3 strongly stained invasive cancer epithelial cells in a near-circumferential pattern (dark staining). In contrast, adjacent normal glandular epithelial cells showed only weak apical positivity (black arrows).

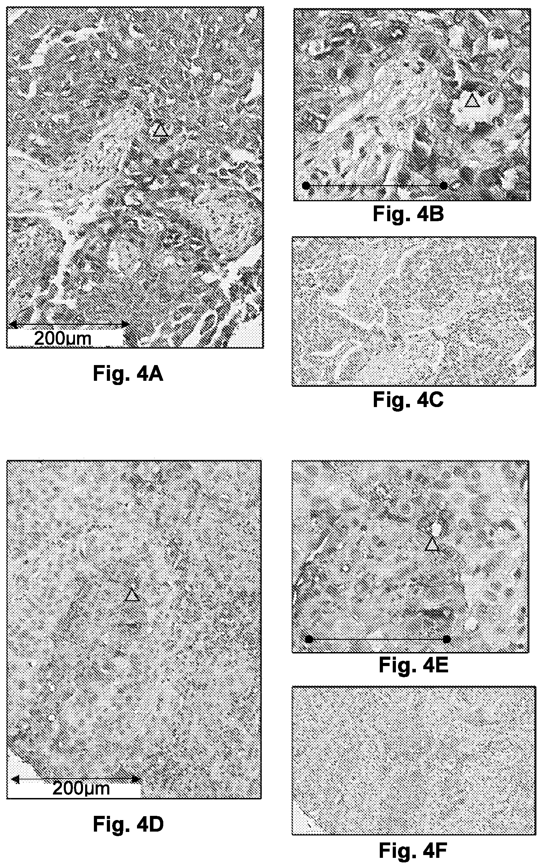

**Figures 4A-4O** show IHC staining of tissue microarrays with anti-MUC1 SEA α-β junction antibody:

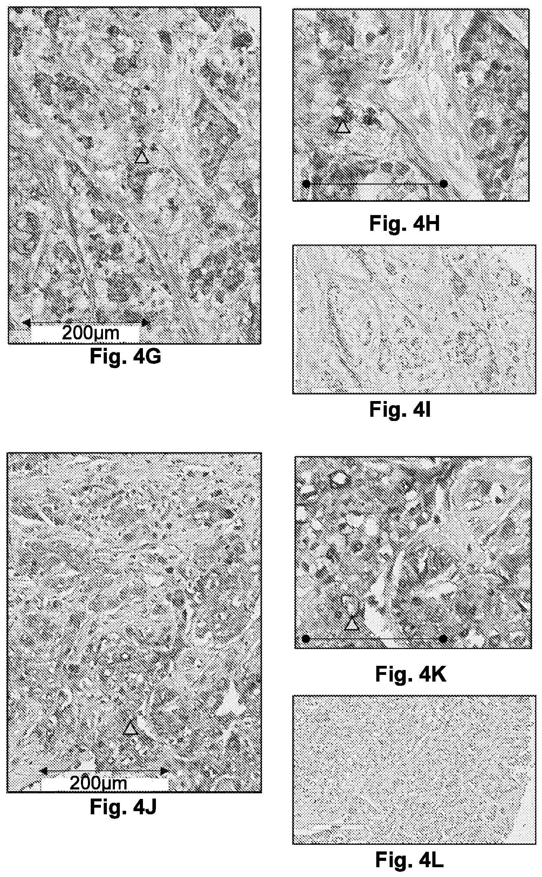

**Figures 4A-4L****:** *IHC of lung, prostate, colon, and breast carcinoma with anti-MUC1 SEA* α-β *junction antibody.* Paraffin embedded microarrays of lung, prostate, colon, and breast carcinomas were stained with antibody DMB5F3 and representative sections are shown in Figures 4A, 4D, 4G and 4J, respectively. Higher magnification is shown in Figures 4B, 4E, 4H and 4K and triangles in Figures 4A, 4D, 4G and 4J and in 4B, 4E, 4H and 4K serve to orientate the magnified region. Staining with antibody DMB5F3 was again abolished in the presence of 100µg/ml of competing soluble MUC1-Xex protein, attesting to the specificity of antibody DMB5F3 (not shown). Control staining with non-specific mouse immunoglobulin is shown in Figures 4C, 4F, 4I and 4L. Antibody DMB5F3 strongly stained malignant cells in a near-circumferential pattern. Lines at the bottom of Figures 4A, 4D, 4G and 4J (with arrows at either ends of the line) and at the bottom of Figures 4B, 4E, 4H and 4K (with full circles at either ends of the line) indicate 200 microns and 100 microns, respectively. Dark staining precipitate indicates MUC1 protein (compare Figures 4A, 4B, 4D, 4E, 4G, 4H, 4J, and 4K with Figures 4C, 4F, 4I and 4L). Tissues in Figures 4A, 4D, 4G and 4J derive from Pathologic Grade 2 lung adenocarcinoma, Pathologic Grade 5 prostate adenocarcinoma, Pathologic Grade 3 colon adenocarcinoma, and infiltrating ductal breast carcinoma, respectively.

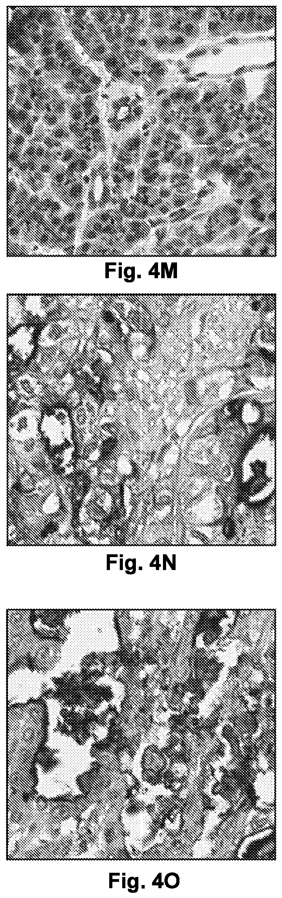

**Figures 4M-4O****:** *Corroborative IHC staining of normal and malignant pancreatic tissues with anti-MUC1 SEA* α-β *junction antibody.* To corroborate high expression and altered molecular architecture of MUC1 in pancreatic carcinoma, additional pancreatic malignancies were stained with anti-MUC1 SEA and compared to normal tissue. Paraffin-embedded microarrays of normal (Figure 4M) and malignant pancreatic tissue (Figures 4N and 4O) were stained with antibody DMB5F3. Staining confirmed weak, apical positivity in normal pancreatic acinar cells (Figure 4M), whereas malignant cells consistently stained in a near-circumferential pattern (Figures 4N and 4O).

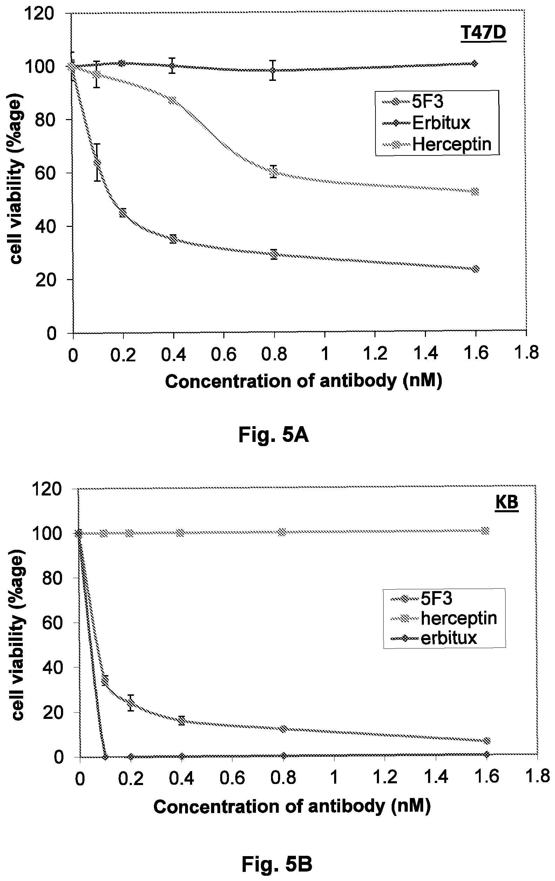

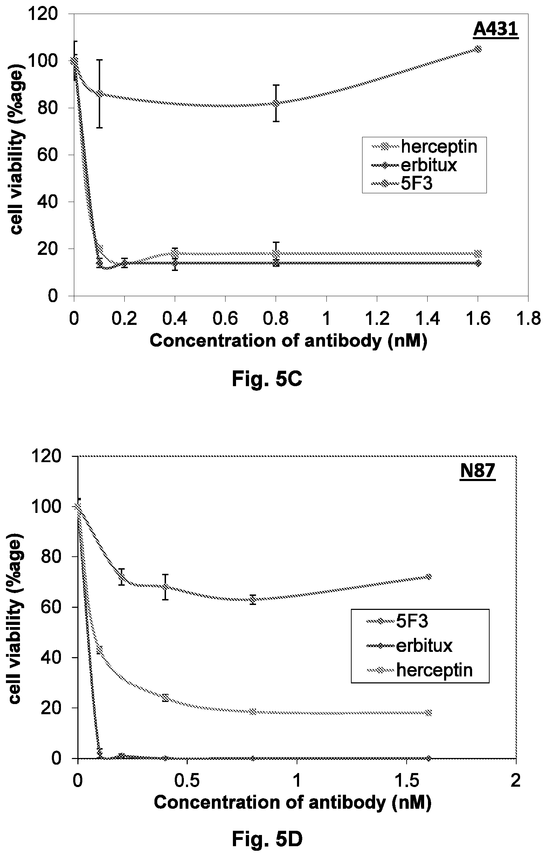

**Figures 5A-5D** are graphs showing comparative cytocidal activity of chDMB5F3, Erbitux, and Herceptin, where each of the three was linked by protein ZZ to the Pseudomonas exotoxin PE38. The three resultant ZZ-PE38 immunotoxins were reacted with human tumor cell lines T47D, KB, A431 and N87 (Fig. 5A-5D, respectively). Immunotoxin chDMB5F3: ZZ-PE38 (oval tracing), Erbitux: ZZ-PE38 (diamond tracing), and Herceptin: ZZ-PE38 (rectangular tracing) were reacted with cells at varying antibody concentrations (x-axis). Cell viability was assessed by the alkaline phosphatase assay (y-axis). Total (100%) viability was determined in control wells to which ZZ-PE38 toxin (5nM) alone had been added.

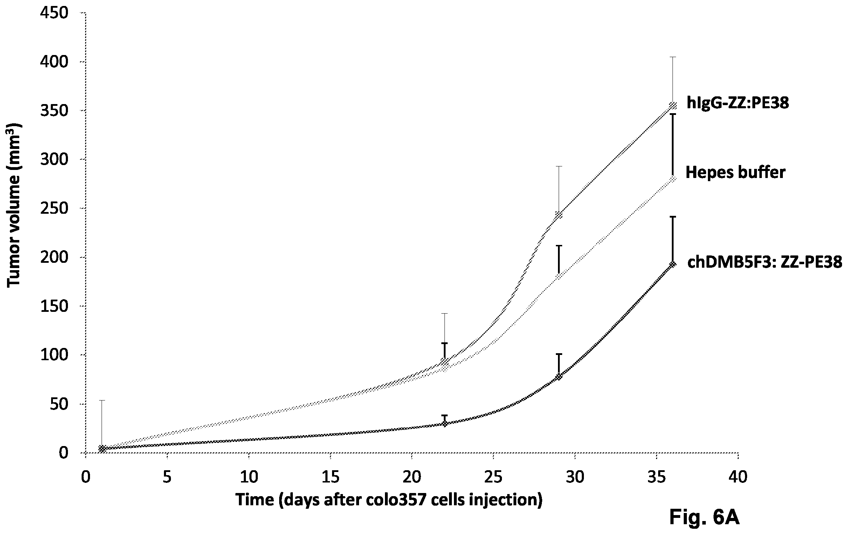

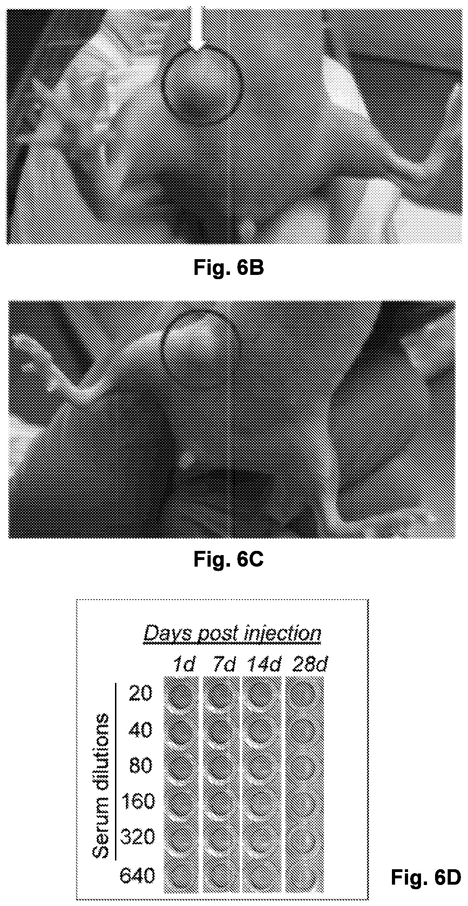

**Figures 6A-6D** show *in vivo* cytotoxicity of chDMB5F3: ZZ-PE38 in human pancreatic tumor xenotransplanted into nude mice. Figure 6A: Nude mice were inoculated with MUC1⁺ pancreatic tumor Colo357 (Day 0). The xenotransplanted mice were then divided into three groups: On Days 1, 6, 9, 14, 22 and 29 Group A received chDMB5F3: ZZ-PE38, Group B received non-specific, isotype-matched IgG-ZZ: PE38 (5µg per injection), while Group C received only Hepes buffer; all administrations were iv (time points indicated by arrows). Tumor volume was measured weekly during the injection period and up to Day 38 in the three groups: mice receiving chDMB5F3: ZZ-PE38 immunotoxin, mice receiving non-specific isotype-matched hIgG-ZZ: PE38, and mice receiving Hepes buffer. Tumor volume in mm³ appears on the y-axis, while photo images show the tumors in the Hepes control mice (Figure 6B) and in mice receiving chDMB5F3: ZZ-PE38 immunotoxin (Figure 6C). Figure 6D: To quantitate serum half-life of the anti-MUC SEA DMB5F3-ZZ-P38 immunotoxin, a single 5 microgram dose was administered to mice, and serum levels were measured by serially diluted Elisa. Compared with Day 7 (7d), serum levels of chDMB5F3 on Days 14 (14d) and 28 (28d) were twofold and fourfold decreased, respectively.

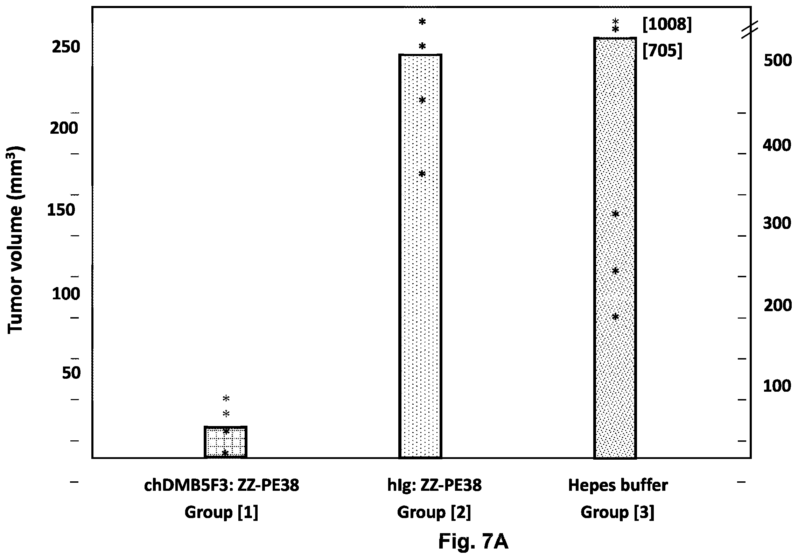

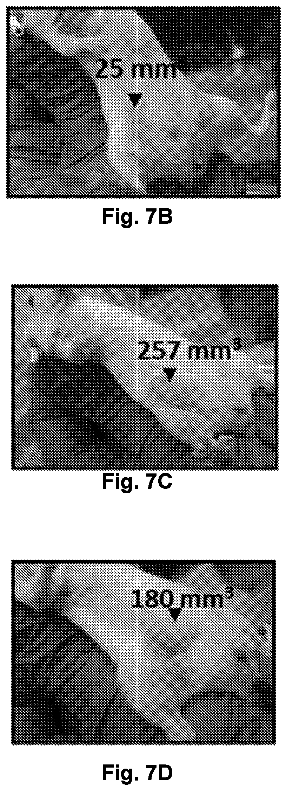

**Figures 7A-7D** show cytotoxicity of chDMB5F3: ZZ-PE38 immunotoxin *in vivo,* demonstrating ablation of MUC1+ pancreatic cancer xenograft in SCID mice. Figure 7A: SCID mice were inoculated subcutaneously with Human Colo357 pancreatic cancer cells on Day 0 and divided into three groups: Group [1] received 5 µg of chDMB5F3: ZZ-PE38 immunotoxin, Group [2] received 5 µg of non-specific matched isotype human Ig: ZZ-PE38 conjugate, and Group [3] Hepes buffer. All xenotransplanted mice of the three groups were injected on Days 1, 4, 8, 11, 16, 24, 31 and 38. Tumor volumes were compared up to 49 days following cell inoculation. The histograms represent the average tumor volumes for each group, while each asterisk represents the value for an individual mouse. The y-axis to the left represents tumor volume (in mm³) for Groups 1 and 2; the y axis to the right represents tumor volumes in Group 3 (the Hepes buffer group) extended to 500 mm³ to include the larger tumor volumes in the Hepes control group. Two points in Group 3 with values above the 500 mm³ point represent tumor volumes of 705 mm³ and 1008 mm³. Figures 7B-7D show representative mice from each group together with their tumor volumes at the conclusion of the 49-day tumor measurement period. Figure 7B: mice which received chDMB5F3: ZZ-PE38 immunotoxin, Figure 7C: mice which received non-specific human Ig:ZZ-PE38 conjugate, and Figure 7D: mice which received only Hepes buffer.

### DETAILED DESCRIPTION OF EMBODIMENTS

The present invention provides sequences of monoclonal antibodies (mAbs) directed against the MUC1 SEA α-β junction (termed the SEA domain), having a robust anti-cancer activity *in vivo.*

The invention provides sequences of antibodies directed against the MUC1 SEA domain, termed herein DMB5F3, and comparative not claimed antibodies, DMB4B4, DMB10F10, DMB4F4, DMB10B7, DMB13D11 and DMC209.

As shown in the Examples below, immunohistochemical staining with the mAb designated DMB5F3 of cells from a series of malignancies including lung, prostate, breast, colon, and pancreatic carcinomas revealed quantitative and qualitative differences between MUC1 expression on normal versus malignant cells: DMB5F3 strongly stained malignant cells in a near-circumferential pattern, whereas MUC1 in normal pancreatic and breast tissue showed only a weak apical pattern of positivity in ductal/acinar cells. Chimeric (mouse-human) DMB5F3 linked to ZZ-PE38 (ZZ being an IgG-binding protein fused to Pseudomonas exotoxin PE38) induced vigorous cytotoxicity of MUC1+ malignant cells *in vitro.* The intensity of cell killing by the anti-MUC1 DMB5F3:ZZ-PE38 protein-exotoxin construct correlated with the level of MUC1 expressed by the target cell, suggesting threshold of MUC1 expression is required for cell killing. In order to demonstrate *in vivo* the effect of antibody DMB5F3 on tumor kill, MUC1+ Colo357 human pancreatic cancer cells were xenotransplanted into nude and SCID mice and then were treated with the chDMB5F3: ZZ-PE38 immunocomplex. In both transplant models (nude and SCID mice), chDMB5F3: ZZ-PE38 demonstrated significant *in vivo* anti-tumor activity, suppressing up to 90% of tumor volume in the SCID mice compared with concomitant controls.

The present invention therefore provides antibodies for use in the treatment of MUC1-expressing malignancies.

Therefore, in a first of its aspects, the present invention provides an isolated monoclonal antibody or antigen-binding fragment thereof which binds to the MUC1 SEA domain.

The term ***"MUC1 SEA domain"*** (also referred to herein as the **"*MUC1 SEA module*"**) refers to a highly conserved domain of 120 amino acids formed by the interaction of the MUC1 α-subunit with the extracellular portion of the MUC1 β-subunit. It is therefore positioned at the MUC1 α-β junction and is a stable cell membrane-fixed moiety; at no time is it shed from the cell surface (see Figures 1A, 1B and 1C, regions indicated by the ovals).

The SEA domain as known in the art is defined as the region located between amino acids 264 and 384 of the human MUC1 protein (UniProtKB - A0A087X2A4 (A0A087X2A4_HUMAN).

The MUC1 transmembrane glycoprotein (MUC-TM) is a heterodimer consisting of an extracellular domain containing between 20 and 125 repeats of a 20 amino acid long sequence (termed the variable number tandem repeat, VNTR), a transmembrane domain, and a short cytoplasmic tail mediating intracellular signaling. MUC1 is auto-proteolytically cleaved within the SEA module. This results in a large extracellular α subunit containing the tandem repeat array bound in a strong non-covalent interaction to a transmembrane β subunit containing the transmembrane and cytoplasmic domains of the molecule.

Specifically, the present invention provides an isolated monoclonal antibody or antigen-binding fragment thereof which binds to MUC1 SEA domain, wherein said antibody comprises:
a. a heavy chain complementarity determining region (CDRH) 1 denoted by SEQ ID NO. 25, CDRH2 denoted by SEQ ID NO. 26, CDRH3 denoted by SEQ ID NO. 27, and a light chain complementarity determining region (CDRL) 1 denoted by SEQ ID NO. 28, a CDRL2 denoted by SEQ ID NO. 29, and a CDRL3 denoted by SEQ ID NO. 30; or comparative not claimed antibodies
b. a heavy chain complementarity determining region (CDRH) 1 denoted by SEQ ID NO. 31, CDRH2 denoted by SEQ ID NO. 32, CDRH3 denoted by SEQ ID NO. 33, and the light chain complementarity determining region (CDRL) 1 denoted by SEQ ID NO. 34, a CDRL2 denoted by SEQ ID NO. 35, and a CDRL3 denoted by SEQ ID NO. 36; or
c. a heavy chain complementarity determining region (CDRH) 1 denoted by SEQ ID NO. 37, CDRH2 denoted by SEQ ID NO. 38, CDRH3 denoted by SEQ ID NO. 39, and the light chain complementarity determining region (CDRL) 1 denoted by SEQ ID NO. 40, a CDRL2 denoted by SEQ ID NO. 41, and a CDRL3 denoted by SEQ ID NO. 42; or
d. a heavy chain complementarity determining region (CDRH) 1 denoted by SEQ ID NO. 43, CDRH2 denoted by SEQ ID NO. 44, CDRH3 denoted by SEQ ID NO. 45, and the light chain complementarity determining region (CDRL) 1 denoted by SEQ ID NO. 46, a CDRL2 denoted by SEQ ID NO. 47, and a CDRL3 denoted by SEQ ID NO. 48; or
e. a heavy chain complementarity determining region (CDRH) 1 denoted by SEQ ID NO. 49, CDRH2 denoted by SEQ ID NO. 50, CDRH3 denoted by SEQ ID NO. 51, and the light chain complementarity determining region (CDRL) 1 denoted by SEQ ID NO. 52, a CDRL2 denoted by SEQ ID NO. 53, and a CDRL3 denoted by SEQ ID NO. 54; or
f. a heavy chain complementarity determining region (CDRH) 1 denoted by SEQ ID NO. 55, CDRH2 denoted by SEQ ID NO. 56, CDRH3 denoted by SEQ ID NO. 57, and the light chain complementarity determining region (CDRL) 1 denoted by SEQ ID NO. 58, a CDRL2 denoted by SEQ ID NO. 59, and a CDRL3 denoted by SEQ ID NO. 60.

As indicated above, the present invention provides isolated monoclonal antibodies that bind to MUC1 SEA domain. The term "***antibody***" refers to a polypeptide encoded by an immunoglobulin gene that specifically binds and recognizes an antigen, in the present case the MUC1 SEA domain.

The term "***monoclonal antibody***", "***monoclonal antibodies***" or "***mAb***" as herein defined refers to a population of homogenous antibodies, i.e., the individual antibodies comprising the population are identical except for possibly naturally occurring rare mutations. Monoclonal antibodies are directed against a single antigenic site (epitope).

Monoclonal antibodies may be prepared and purified by any method known in the art. For example, monoclonal antibodies may be prepared from B cells taken from the spleen or lymph nodes of immunized animals (e.g., rabbits, rats, mice, or monkeys).

Purification of monoclonal antibodies may be performed using any method known in the art, for example by affinity chromatography, namely, by using an affinity column to which a specific epitope (or antigen) is conjugated. Alternatively, purification of antibodies may be based on using protein A and protein G column chromatography.

An exemplary antibody structural unit comprises a tetramer, as known in the art. Each tetramer is composed of two identical pairs of polypeptide chains, each pair having one "***light chain***" and one "***heavy chain***". The N-terminus of each chain defines a variable region of about 100 to 110 or more amino acids primarily responsible for antigen (epitope) recognition.

Thus, the terms "***heavy chain variable region***" (V_{H}) and "***light chain variable region***" (V_{L}) refer to these heavy and light chains, respectively. More specifically, the variable region is subdivided into hypervariable and framework (FR) regions. Hypervariable regions have a high ratio of different amino acids in a specific position, relative to the most common amino acid in that position. Four FR regions which have more stable amino acids sequences separate the hypervariable regions. The hypervariable regions directly contact a portion of the antigen's surface. For this reason, hypervariable regions are herein referred to as "***complementarity determining regions***", or "***CDRs***", the CDRs are positioned both at the heavy chain of the antibody (a "***heavy chain complementarity determining region***") and at the light chain of the antibody (a "***light chain complementarity determining region***").

From N-terminal to C-terminal, both light and heavy chains comprise the domains FR1, CDR1, FR2, CDR2, FR3, CDR3 and FR4. The CDRs are primarily responsible for binding to an epitope of an antigen. The CDRs of each chain are typically referred to as CDR1, CDR2, and CDR3, numbered sequentially starting from the N-terminus, and are also typically identified by the chain in which the CDR is located.

Thus, the complementarity determining regions CDRH1, CDRH2 and CDRH3 refer to the three complementarity determining regions starting from the N-terminus of the antibody's heavy chain (also referred to herein as heavy chain complementarity determining region) and the complementarity determining regions CDRL1, CDRL2 and CDRL3 refer to the three complementarity determining regions starting from the N-terminus of the antibody's light chain (also referred to herein as light chain complementarity determining region).

The present invention encompasses antigen-binding fragments of the isolated anti MUC1 SEA domain monoclonal antibody of the invention.

As used herein the term ***"antigen binding fragment"*** relates to a fragment of the full length antibody which retains the antibody's specificity of binding to MUC1 SEA domain. An antigen binding fragment encompasses but is not limited to Fv, single chain Fv (scFv), single chain Fv-Fc (scFv-Fc), Fab', Fab, F(ab')₂ and F(ab)₂.

Such fragments may be produced by any method known in the art, for example by proteolytic cleavage, using enzymes such as papain (to produce Fab fragments) or pepsin (to produce F(ab')₂ fragments).

Thus, in some embodiments the antibody according to the invention is wherein said antibody is an antibody fragment selected from the group consisting of a single-chain Fv-Fc (scFv-Fc) molecule, single chain Fv (scFv), Fv, Fab', Fab, F(ab')₂, and F(ab)₂.

In specific embodiments the isolated monoclonal antibody of the invention or the antigen-binding fragment thereof binds to the MUC1 SEA domain.

As demonstrated in Example 5 below, administration of an immunocomplex comprising the antibody DMB5F3 and a cytotoxic moiety significantly reduced tumor volume in an *in vivo* cancer model in mice. Therefore, in specific embodiments the isolated monoclonal antibodies of the invention or the antigen-binding fragment thereof are effective in reducing tumor volume in a subject.

By the term "***reducing***" tumor volume in the context of the present invention it is meant that the isolated monoclonal antibody of the invention or the antigen-binding fragment thereof lowers the size of the tumor as measured by any means known in the art, by at least about 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or about 100% as compared to the tumor size in the absence of the antibody of the invention or the antigen-binding fragment thereof. In certain embodiments the term "reducing" is meant to refer to a reduction of at least about 50%, 60%, 70%, 80%, or 90%.

In some embodiments the isolated anti-MUC1 SEA domain monoclonal antibody is a murine antibody, a chimeric antibody, a humanized antibody, or a human antibody.

The term "***chimeric antibody***" as used herein refers to an antibody that possesses antigen-binding variable domains from one species (e.g., the variable domains of a murine antibody) and constant domains of a different species (e.g., human).

The term "***humanized antibody***" as used herein refers to an antibody that is based on the structure of a non-human species (e.g., a mouse) whose amino acid sequence has been modified to increase its similarity to antibody variants produced naturally in humans.

The term "***human antibody***" as used herein refers to an antibody that possesses an amino acid sequence corresponding to that of an antibody produced by a human and/or has been made using any of the techniques for making human antibodies known in the art. This definition specifically excludes a humanized antibody that comprises non-human antigen-binding residues.

Methods for the preparation of chimeric, humanized, and human antibodies are well known in the art.

For preparing large quantities of the antibody (either chimeric, humanized, or human), a stable cell line expressing the antibody can be prepared, by transfecting cells (e.g., CHO cells) with the Ig expression vector containing both heavy and light chains of the antibody. The antibodies may then be manufactured for example in a bioreactor system. The antibodies may be purified to clinical grade using well established monoclonal antibody purification methods. Clones producing high levels of anti-MUC1 SEA domain antibody may then be selected and expanded based on antibody levels in the supernatant, as tested by any method known in the art, for example, a MUC1 SEA domain-specific ELISA assay. A master cell bank developed for the specific clone may serve as the starting growing material for all clinical grade batches.

In some embodiments, the present invention provides an anti-MUC1 SEA domain isolated monoclonal antibody or antigen-binding fragment thereof, wherein said antibody or antigen-binding fragment thereof comprises a heavy chain variable region and a light chain variable region, wherein said heavy chain variable region is encoded by a nucleic acid sequence which is at least 70%, or 75%, or 80%, or 85%, or 90% or more identical to the nucleic acid sequence denoted by SEQ ID NO. 1 and wherein said light chain variable region is encoded by a nucleic acid sequence which is at least 70%, or 75%, or 80%, or 85%, or 90% or more identical to the nucleic acid sequence denoted by SEQ ID NO. 2.

In other embodiments, the present invention provides a comparative not claimed anti-MUC1 SEA domain isolated monoclonal antibody or antigen-binding fragment thereof, wherein said antibody or antigen-binding fragment thereof is comprised of a heavy chain variable region and a light chain variable region, wherein said heavy chain variable region is encoded by a nucleic acid sequence which is at least 70%, or 75%, or 80%, or 85%, or 90% or more identical to the nucleic acid sequence denoted by SEQ ID NO. 3 and wherein said light chain variable region is encoded by a nucleic acid sequence which is at least 70%, or 75%, or 80%, or 85%, or 90% or more identical to the nucleic acid sequence denoted by SEQ ID NO. 4.

In some further embodiments, the present invention provides a comparative not claimed anti-MUC1 SEA domain isolated monoclonal antibody or antigen-binding fragment thereof, wherein said antibody or antigen-binding fragment thereof is comprised of a heavy chain variable region and a light chain variable region, wherein said heavy chain variable region is encoded by a nucleic acid sequence which is at least 70%, or 75%, or 80%, or 85%, or 90% or more identical to the nucleic acid sequence denoted by SEQ ID NO. 5 and wherein said light chain variable region is encoded by a nucleic acid sequence which is at least 70%, or 75%, or 80%, or 85%, or 90% or more identical to the nucleic acid sequence denoted by SEQ ID NO. 6.

In some embodiments, the present invention provides a comparative not claimed anti MUC1-SEA domain isolated monoclonal antibody or antigen-binding fragment thereof, wherein said antibody or antigen-binding fragment thereof is comprised of a heavy chain variable region and a light chain variable region, wherein said heavy chain variable region is encoded by a nucleic acid sequence which is at least 70%, or 75%, or 80%, or 85%, or 90% or more identical to the nucleic acid sequence denoted by SEQ ID NO. 7 and wherein said light chain variable region is encoded by a nucleic acid sequence which is at least 70%, or 75%, or 80%, or 85%, or 90% or more identical to the nucleic acid sequence denoted by SEQ ID NO. 8.

In some embodiments, the present invention provides a comparative not claimed anti-MUC1 SEA domain isolated monoclonal antibody or antigen-binding fragment thereof, wherein said antibody or antigen-binding fragment thereof comprises a heavy chain variable region and a light chain variable region, wherein said heavy chain variable region is encoded by a nucleic acid sequence which is at least 70%, or 75%, or 80%, or 85%, or 90% or more identical to the nucleic acid sequence denoted by SEQ ID NO. 9 and wherein said light chain variable region is encoded by a nucleic acid sequence which is at least 70%, or 75%, or 80%, or 85%, or 90% or more identical to the nucleic acid sequence denoted by SEQ ID NO. 10.

In some embodiments, the present invention provides a comparative not claimed anti-MUC1 SEA domain isolated monoclonal antibody or antigen-binding fragment thereof, wherein said antibody or antigen-binding fragment thereof comprises a heavy chain variable region and a light chain variable region, wherein said heavy chain variable region is encoded by a nucleic acid sequence which is at least 70%, or 75%, or 80%, or 85%, or 90% or more identical to the nucleic acid sequence denoted by SEQ ID NO. 11 and wherein said light chain variable region is encoded by a nucleic acid sequence which is at least 70%, or 75%, or 80%, or 85%, or 90% or more identical to the nucleic acid sequence denoted by SEQ ID NO. 12.

In some embodiments, the present invention provides an anti-MUC1 SEA domain isolated monoclonal antibody or antigen-binding fragment thereof, wherein said antibody or antigen-binding fragment thereof comprises a heavy chain variable region comprising the amino acid sequence denoted by SEQ ID NO. 13 and a light chain variable region comprising the amino acid sequence denoted by SEQ ID NO. 14.

In other embodiments, the present invention provides a comparative not claimed anti-MUC1 SEA domain isolated monoclonal antibody or antigen-binding fragment thereof, wherein said antibody or antigen-binding fragment thereof comprises a heavy chain variable region comprising the amino acid sequence denoted by SEQ ID NO. 15 or a variant thereof and a light chain variable region comprising the amino acid sequence denoted by SEQ ID NO. 16, or a variant thereof.

In some further embodiments, the present invention provides a comparative not claimed anti-MUC1 SEA domain isolated monoclonal antibody or antigen-binding fragment thereof, wherein said antibody or antigen-binding fragment thereof comprises a heavy chain variable region comprising the amino acid sequence denoted by SEQ ID NO. 17 or a variant thereof and a light chain variable region comprising the amino acid sequence denoted by SEQ ID NO. 18, or a variant thereof.

In some embodiments, the present invention provides a comparative not claimed anti-MUC1 SEA domain isolated monoclonal antibody or antigen-binding fragment thereof, wherein said antibody or antigen-binding fragment thereof comprises a heavy chain variable region comprising the amino acid sequence denoted by SEQ ID NO. 19 or a variant thereof and a light chain variable region comprising the amino acid sequence denoted by SEQ ID NO. 20, or a variant thereof.

In certain embodiments, the present invention provides a comparative not claimed anti-MUC1 SEA domain isolated monoclonal antibody or antigen-binding fragment thereof, wherein said antibody or antigen-binding fragment thereof comprises a heavy chain variable region comprising the amino acid sequence denoted by SEQ ID NO. 21 or a variant thereof and a light chain variable region comprising the amino acid sequence denoted by SEQ ID NO. 22, or a variant thereof.

In some embodiments, the present invention provides a comparative not claimed anti-MUC1 SEA domain isolated monoclonal antibody or antigen-binding fragment thereof, wherein said antibody or antigen-binding fragment thereof comprises a heavy chain variable region comprising the amino acid sequence denoted by SEQ ID NO. 23 or a variant thereof and a light chain variable region comprising the amino acid sequence denoted by SEQ ID NO. 24, or a variant thereof.

In other embodiments the isolated antibody according to the invention is wherein said antibody is an anti MUC1 SEA domain isolated monoclonal antibody or antigen-binding fragment thereof, wherein said antibody comprises six CDR sequences as denoted by SEQ ID Nos. 25-30, and a heavy chain variable region comprising an amino acid sequence having at least 90% sequence identity to SEQ ID NO. 13 and a light chain variable region comprising an amino acid sequence having at least 90% sequence identity to SEQ ID NO: 14.

In other embodiments the isolated antibody is an anti MUC1 SEA domain isolated monoclonal antibody or antigen-binding fragment thereof, wherein said antibody comprises six CDR sequences as denoted by SEQ ID Nos. 31-36, and a heavy chain variable region comprising an amino acid sequence having at least 90% sequence identity to SEQ ID NO:15 and a light chain variable region comprising an amino acid sequence having at least 90% sequence identity to SEQ ID NO: 16.

In further embodiments the isolated antibody is an anti MUC1 SEA domain isolated monoclonal antibody or antigen-binding fragment thereof, wherein said antibody comprises six CDR sequences as denoted by SEQ ID Nos. 37-42, and a heavy chain variable region comprising an amino acid sequence having at least 90% sequence identity to SEQ ID NO. 17 and a light chain variable region comprising an amino acid sequence having at least 90% sequence identity to SEQ ID NO. 18.

In various embodiments is an anti MUC1 SEA domain isolated monoclonal antibody or antigen-binding fragment thereof, wherein said antibody comprises six CDR sequences as denoted by SEQ ID Nos. 43-48, and a heavy chain variable region comprising an amino acid sequence having at least 90% sequence identity to SEQ ID NO. 19 and a light chain variable region comprising an amino acid sequence having at least 90% sequence identity to SEQ ID NO. 20.

In other embodiments the isolated antibody is an anti MUC1 SEA domain isolated monoclonal antibody or antigen-binding fragment thereof, wherein said antibody comprises six CDR sequences as denoted by SEQ ID Nos. 49-54, and a heavy chain variable region comprising an amino acid sequence having at least 90% sequence identity to SEQ ID NO. 21 and a light chain variable region comprising an amino acid sequence having at least 90% sequence identity to SEQ ID NO. 22.

In further embodiments the isolated antibody is an anti MUC1 SEA domain isolated monoclonal antibody or antigen-binding fragment thereof, wherein said antibody comprises six CDR sequences as denoted by SEQ ID Nos. 55-60, and a heavy chain variable region comprising an amino acid sequence having at least 90% sequence identity to SEQ ID NO. 23 and a light chain variable region comprising an amino acid sequence having at least 90% sequence identity to SEQ ID NO: 24.

The nucleic acid sequences encoding the heavy and light chains of the antibodies termed herein DMB5F3, DMB7F3, DMB4B4, DMB4F4, DMB 10B7, and DMC209 are detailed in Table 1 below. In addition, the amino acid sequences of the heavy and light chains of the antibodies described herein are detailed in Table 2 below. Furthermore, the sequences of the CDRs of the above antibodies are displayed in Table 3 below.

DMB4B4 and DMB10F10 have identical amino acid sequences.

DMB4F4 is mIg-gamma1. DMB10B7 and DMB13D11 are mIgA. However, the variable regions of all three antibodies: DMB4F4, DMB10B7 and DMB13D11 are identical.

**Table 1: Nucleic acid sequences**

| SEQ ID NO. | Sequence | Description |
|---|---|---|
| 1 | ATGAGAGTGCTGATTCTTTTGTGCCTGTTCACAGCCTT TCCTGGTGTCCTGTCTGATGTGCAGGTTCAGGAGTCAG GACCTGACCTGGTGAAACCTTCTCAGTCACTTTCTCTC ACCTGCACTGTCACTGGCCACTCCATCACCAGAGGTTC TAGCTGGCACTGGATCCGGCAGTTTCCAGGAAACAAAC TGGAGTGGATGGGATACATACATTACGGTGGTGGCACT TCCTACAACCCATCTCTCAAAAGTCGAATCTCTATCAC TCGAGACACATCCAAGAACCAGTTCTTCCTGCAGTTGA ATTCTGTGACTACTGAGGACACAGCCACATTTTTCTGT GCACGGTATTCCTACGATATTACCTACCGCTGGTTCTT CGATGTCTGGGGCGCAGGGACCACGGTCATCGTCTCCT | DMB5F3 Heavy chain nucleic acid sequence |
| | CA | |
| 2 | ATGGTATCCACACCTCAGTTCCTTGTATTTTTGCTTTT CTGGATTCCAGCCTCCAGAGGTGACATCTTGCTGACTC AGTCTCCAGCCATCCTGTCTGTGAGTCCAGGAGAAAGA GTCAGTTTCTCCTGCAGGGCCAGTCAGAACATTGGCAC AAGCATACACTGGTATCAGCAAAGAAAAAATGGTTCTC CAAGACTTCTCATAAAGTATGCTTCTGAGTCTATCTCT GGGATTCCTTCCAGGTTTAGTGGCAGCGGATCTGGGAC AGATTTTACTCTTTCCATCAACAGTGTGGAGTCTGAAG ATATGGCAGATTATTACTGTCAACAAAATAATAACTGG CCGCTCACGTTCGGTGCTGGGACCAAGCTGGAACTGAA A | DMB5F3 Light chain nucleic acid sequence |
| 3 | ATGGAATGGCCTTGTATCTTTCTCTTCCTCCTGTCAGT AACTGAAGGTGTCCACTCCCAGGTTCACCTTCAGCAGT CTGGGGCTGAGCTGGTGAGGCCTGGGTCCTCAGTGAAG ATTTCCTGTAAGGCTTCTGGCTATGAATTCAATAGTTT CTGGATGAACTGGGTGAAGCAGAGGCCTGGACAGGGTC TTGAGTGGATTGGACAGATTTATCCTGGAGATGGTGAT ACTAACTACAATGGAAAATTCAAGGGTAAAGCCACACT GACTGCTGACAAATCCTCCAGCACAGCCTACATGCAGC TCAGCAGCCTAACATCTGAGGACTCTGGGATTTACTTC TGTGCAAGAGGATACAAGGCCTGGTTTATTTACTGGGG CCAAGGGACTCTGGTCACTGTCTCTGAA | DMB4B4 Heavy chain nucleic acid sequence |
| 4 | ATGGTATCCACACCTCAGTTCCTTGTATTTTTGCTTTT CTGGATTCCAGCCTCCAGAGGTGACGTCTTGCTGACTC AGTCTCCAGCCATTCTGTCTGTGAGTCCAGGAGAAAGA GTCAGTTTCTCCTGCAGGGCCAGTCAGAACATTGGCAC AAGCATACACTGGTATCAGCAAAGCACAAATGGTTCTC CAAGGCTTATCATAAAGTATGCTTCTGAGTCTCTCTCT GGGATCCCTTCCAGGTTTAGTGGCAGTGGATCAGGGAC AGATTTTACTCTTACCATCAACAGTGTGGAGTCTGAGG ATATTGCAGATTATTACTGTCAACAAAGTAATGGCTGG CCGCTCACGTTCGGTGGTGGGACCAAGCTGGAGCTGAA A | DMB4B4 Light chain nucleic acid sequence |
| 5 | ATGGGATGGAGCTGGATCTTTCTCTTCCTCCTGTCAGG AACTGCAGGTGTCCTCTCTGAGGTCCAGCTGCAACAGT CTGGACCTGAACTGGTGAAGCCTAAAACTTCAATGAAG | DMB4F4 Heavy chain nucleic acid sequence |
| | ATATCCTGCAAGGCCTCTGGTTACTCATTCACTGACTT CACCATGAACTGGGTGAAGCAGAGCCATGGAAAAAACC CTGAGTGGATTGGACTTATTACTCCTTACAATGGTGGA ACTAGTTACAACCAGAAGTTCAAGGGCAAGGCCACATT TACTGTAGACAGGTCATCCAGCACTGCCTACATGGAAC TCCTCAGTCTGACATCTGAGGACTCTGCGGTCTATTAC TGTGCAAGAGGCCTGACCTATTTTGACCAGTGGGGCCA AGGCACCACTCTCACAGTCTCCTCA | |
| 6 | ATGGATTTTCAAGTGCAGATTTTCAGCTTCCTGCTAAT GAGTGCCTCAGTCATAATGTCCAGGGGACAAATTGTTC TCACCCAGTCTCCAGCACTCATGTCTGCATCTCCAGGG GAGAAGGTCACCATGACCTGCAGTGCCAGCTCAAGTGT AAGTTACATGTACTGGTATCAGCAGAAGCCAACATCCT CCCCCAAACCCTGGATTTTGCTCACTTCCAACCTGGCT TCTGGAGTCCCTACTCGCTTCAGTGGCAGTGGGTCTGG GACCTCTTACTCTCTCACAATCAGCAGCATGGAGGCTG AAGATGCTGCCACTTATTACTGCCAGCAGTGGAATAGT AAACCACCCATCACGTTCGGAGGGGGGACCAAGCTGGA AATAAAA | DMB4F4 Light chain nucleic acid sequence |
| 7 | ATGGGATGGAGCTGGATCTTTCTCTTCCTCCTGTCAGG AACTGCAGGTGTCCTCTCTGAGGTCCAGCTGCAACAGT CTGGACCTGAACTGGTGAAGCCTAAAACTTCAATGAAG ATATCCTGCAAGGCCTCTGGTTACTCATTCACTGACTT CACCATGAACTGGGTGAAGCAGAGCCATGGAAAAAACC CTGAGTGGATTGGACTTATTACTCCTTACAATGGTGGA ACTAGTTACAACCAGAAGTTCAAGGGCAAGGCCACATT TACTGTAGACAGGTCATCCAGCACTGCCTACATGGAAC TCCTCAGTCTGACATCTGAGGACTCTGCGGTCTATTAC TGTGCAAGAGGCCTGACCTATTTTGACCAGTGGGGCCA AGGCACCACTCTCACAGTCTCCTCA | DMB10B7 Heavy chain nucleic acid sequence |
| 8 | ATGGATTTTCAAGTGCAGATTTTCAGCTTCCTGCTAAT GAGTGCCTCAGTCATAATGTCCAGGGGACAAATTGTTC TCACCCAGTCTCCAGCACTCATGTCTGCATCTCCAGGG GAGAAGGTCACCATGACCTGCAGTGCCAGCTCAAGTGT AAGTTACATGTACTGGTATCAGCAGAAGCCAACATCCT CCCCCAAACCCTGGATTTTGCTCACTTCCAACCTGGCT TCTGGAGTCCCTACTCGCTTCAGTGGCAGTGGGTCTGG GACCTCTTACTCTCTCACAATCAGCAGCATGGAGGCTG AAGATGCTGCCACTTATTACTGCCAGCAGTGGAATAGT AAACCACCCATCACGTTCGGAGGGGGGACCAAGCTGGA AATAAAA | DMB10B7 Light chain nucleic acid sequence |
| 9 | ATGGCTGTCCTGGGGCTGCTTCTCTGCCTGGTGACGTT CCCAAGCTGTGTCCTGTCCCAGGTGCAGCTGAAGGAAT CAGGACCTGGCCTAGTGGCGCCCTCACAGAACCTGTCC | DMB7F3 Heavy chain nucleic acid sequence |
| | ATCACATGCACTGTCTCAGGTTTCTCATTAACCGACTA TGGTGTAAACTGGGTTCGCCAGCCTTCAGGAAAGGGTC TGGAGTGGCTGGGAGAAATATGGGCTGGTGGAACTACA TTCTATAATTCAGCTCTCAAATCCAGACTGACCATCAC CAAGGACAACTCCAAGAGCCAAGTTTTCTTAGAAATGA ACAGTCTGCAAAGTCATGACACAGCCATGTACTATTGT GCCAAACGGCTTAACTGGGACAGTTCTATGGACTACTG GGGTCAAGGAACCTCAGTCACCGTCTCCTCA | |
| 10 | ATGGAGACAGACACACTCCTGTTATGGGTACTGCTGCT CTGGGTTCCAGGTTCCACTGGTGACATTGTGCTGACAC AGTCTCCTGCTTCCTTTGCTGTATCTCTGGGACAGAGG GCCACCATCTCATGCAGGGCCAGCGAAAGTGTCAGTAC GTCTGCCTATAATTTTCTGCACTGGTACCAGCAGAAAC CTGGACAGCCACCCAAACTCCTCATCTATCTTGCATCC AACCTAGAATCTGGGGTCCCTGCCAGGTTCAGTGGCAG TGGGTCTGGGACAGACTTCACCCTCAACATCCATCCTG TGGAGGAGGAGGATGCTGCAACCTATTACTGTCAGCAC AGTAGGGAGCTTCCGTACACGTTCGGAGGGGGGACCAA GTTGGAAATAAAA | DMB7F3 Light chain nucleic acid sequence |
| 11 | | DMC209 Heavy chain nucleic acid sequence |
| 12 | | DMC209 Light chain nucleic acid sequence |

**Table 2: Amino acid sequences**

| SEQ ID NO. | Sequence | Description |
|---|---|---|
| 13 | MRVLILLCLFTAFPGVLSDVQVQESGPDLVKPSQSLSL TCTVTGHSITRGSSWHWIRQFPGNKLEWMGYIHYGGGT SYNPSLKSRISITRDTSKNQFFLQLNSVTTEDTATFFC ARYSYDITYRWFFDVWGAGTTVIVSS | DMB5F3 Heavy chain amino acid sequence |
| 14 | MVSTPQFLVFLLFWIPASRGDILLTQSPAILSVSPGER VSFSCRASQNIGTSIHWYQQRKNGSPRLLIKYASESIS GIPSRFSGSGSGTDFTLSINSVESEDMADYYCQQNNNW PLTFGAGTKLELK | DMB5F3 Light chain amino acid sequence |
| 15 | MEWPCIFLFLLSVTEGVHSQVHLQQSGAELVRPGSSVK ISCKASGYEFNSFWMNWVKQRPGQGLEWIGQIYPGDGD TNYNGKFKGKATLTADKSSSTAYMQLSSLTSEDSGIYF CARGYKAWFIYWGQGTLVTVSE | DMB4B4 Heavy chain amino acid sequence |
| 16 | MVSTPQFLVFLLFWIPASRGDVLLTQSPAILSVSPGER VSFSCRASQNIGTSIHWYQQSTNGSPRLIIKYASESLS GIPSRFSGSGSGTDFTLTINSVESEDIADYYCQQSNGW PLTFGGGTKLELK | DMB4B4 Light chain amino acid sequence |
| 17 | MGWSWIFLFLLSGTAGVLSEVQLQQSGPELVKPKTSMK ISCKASGYSFTDFTMNWVKQSHGKNPEWIGLITPYNGG TSYNQKFKGKATFTVDRSSSTAYMELLSLTSEDSAVYY CARGLTYFDQWGQGTTLTVSS | DMB4F4 Heavy chain amino acid sequence |
| 18 | MDFQVQIFSFLLMSASVIMSRGQIVLTQSPALMSASPG EKVTMTCSASSSVSYMYWYQQKPTSSPKPWILLTSNLA SGVPTRFSGSGSGTSYSLTISSMEAEDAATYYCQQWNS KPPITFGGGTKLEIK | DMB4F4 Light chain amino acid sequence |
| 19 | MGWSWIFLFLLSGTAGVLSEVQLQQSGPELVKPKTSMK ISCKASGYSFTDFTMNWVKQSHGKNPEWIGLITPYNGG TSYNQKFKGKATFTVDRSSSTAYMELLSLTSEDSAVYY CARGLTYFDQWGQGTTLTVSS | DMB10B7 Heavy chain amino acid sequence |
| 20 | MDFQVQIFSFLLMSASVIMSRGQIVLTQSPALMSASPG EKVTMTCSASSSVSYMYWYQQKPTSSPKPWILLTSNLA SGVPTRFSGSGSGTSYSLTISSMEAEDAATYYCQQWNS KPPITFGGGTKLEIK | DMB10B7 Light chain amino acid sequence |
| 21 | MAVLGLLLCLVTFPSCVLSQVQLKESGPGLVAPSQNLS ITCTVSGFSLTDYGVNWVRQPSGKGLEWLGEIWAGGTT FYNSALKSRLTITKDNSKSQVFLEMNSLQSHDTAMYYC AKRLNWDSSMDYWGQGTSVTVSS | DMB7F3 Heavy chain amino acid sequence |
| 22 | METDTLLLWVLLLWVPGSTGDIVLTQSPASFAVSLGQR ATISCRASESVSTSAYNFLHWYQQKPGQPPKLLIYLAS NLESGVPARFSGSGSGTDFTLNIHPVEEEDAATYYCQH SRELPYTFGGGTKLEIK | DMB7F3 Light chain amino acid sequence |
| 23 | | DMC209 Heavy chain amino acid sequence |
| 24 | | DMC209 Light chain amino acid sequence |

The CDR sequences are highlighted within the heavy and the light chain amino acid sequences and are listed in Table 3.

**Table 3: CDR amino acid sequences**

| SEQ ID NO. | Sequence | Description |
|---|---|---|
| 25 | RGSSWH | DMB5F3 Heavy chain CDR H1 |
| 26 | YIHYGGGTSYNPSLKS | DMB5F3 Heavy chain CDR H2 |
| 27 | YSYDITYRWFFDV | DMB5F3 Heavy chain CDR H3 |
| 28 | RASQNIGTSIH | DMB5F3 Light chain CDR L1 |
| 29 | YASESIS | DMB5F3 Light chain CDR L2 |
| 30 | QQNNNWPLT | DMB5F3 Light chain CDR L3 |
| 31 | SFWMN | DMB4B4 Heavy chain CDR H1 |
| 32 | QIYPGDGDTNYNGKFKG | DMB4B4 Heavy chain CDR H2 |
| 33 | GYKAWFIY | DMB4B4 Heavy chain CDR H3 |
| 34 | RASQNIGTSIH | DMB4B4 Light chain CDR L1 |
| 35 | YASESLS | DMB4B4 Light chain CDR L2 |
| 36 | QQSNGWPLT | DMB4B4 Light chain CDR L3 |
| 37 | DFTMN | DMB4F4 Heavy chain CDR H1 |
| 38 | LITPYNGGTSYNQKFKG | DMB4F4 Heavy chain CDR H2 |
| 39 | GLTYFDQ | DMB4F4 Heavy chain CDR H3 |
| 40 | SASSSVSYMY | DMB4F4 Light chain CDR L1 |
| 41 | LTSNLAS | DMB4F4 Light chain CDR L2 |
| 42 | QQWNSKPPIT | DMB4F4 Light chain CDR L3 |
| 43 | DFTMN | DMB10B7 Heavy chain CDR H1 |
| 44 | LITPYNGGTSYNQKFKG | DMB10B7 Heavy chain CDR H2 |
| 45 | GLTYFDQ | DMB10B7 Heavy chain CDR H3 |
| 46 | SASSSVSYMY | DMB10B7 Light chain CDR L1 |
| 47 | LTSNLAS | DMB10B7 Light chain CDR L2 |
| 48 | QQWNSKPPIT | DMB10B7 Light chain CDR L3 |
| 49 | DYGVN | DMB7F3 Heavy chain CDR H1 |
| 50 | EIWAGGTTFYNSALKS | DMB7F3 Heavy chain CDR H2 |
| 51 | RLNWDSSMDY | DMB7F3 Heavy chain CDR H3 |
| 52 | RASESVSTSAYNFLH | DMB7F3 Light chain CDR L1 |
| 53 | LASNLES | DMB7F3 Light chain CDR L2 |
| 54 | QHSRELPYT | DMB7F3 Light chain CDR L3 |
| 55 | | DMC209 Heavy chain CDR H1 |
| 56 | | DMC209 Heavy chain CDR H2 |
| 57 | | DMC209 Heavy chain CDR H3 |
| 58 | | DMC209 Light chain CDR L1 |
| 59 | | DMC209 Light chain CDR L2 |
| 60 | | DMC209 Light chain CDR L3 |

The genomic derivation of the antibodies is shown in the following Tables.

**Table 4: Group I, DMBSF3**

| Sequence | V-gene and allele | V-region identity % (nt) | J-gene and allele | D-gene and allele | AA |
|---|---|---|---|---|---|
| | | | | | Junction |
| V_{H} | Musmus IGHV3-1*02F | 95.14% (274/288nt) | Musmus IGHJ1*01F | Musmus IGHD1-1*01F | CARYSYDI |
| | | | | | TYRWFFDVW |
| V_{L} | Musmus IGKV5-48*01F | 96.06% (268/279nt) | Musmus IGKJ5*01F | --- | CQQNNN |
| | | | | | WPLTF |

**Table 5: Group II, DMB7F3**

| Sequence | V-gene and allele | V-region identity % (nt) | J-gene and allele | D-gene and allele | AA |
|---|---|---|---|---|---|
| | | | | | Junction |
| V_{H} | Musmus IGHV2-6-5*01F | 93.68% (267/285nt) | Musmus IGHJ4*01F | Musmus IGHD4-1*01F | CAKRLNW |
| | | | | | DSSMDYW |
| V_{L} | Musmus IGKV3-12*01F | 96.56% (281/291nt) | Musmus IGKJ2*01F | --- | CQHSREL |
| | | | | | PYTF |

**Table 6: Group III, DMB4B4, DMB10F10**

| Sequence | V-gene and allele | V-region identity % (nt) | J-gene and allele | D-gene and allele | AA |
|---|---|---|---|---|---|
| | | | | | Junction |
| V_{H} | Musmus IGHV1-80*01F | 92.01% (265/288nt) | Musmus IGHJ3*01F | Musmus IGHD1-2*01F | CARGYKA |
| | | | | | WFIYW |
| V_{L} | Musmus IGKV5-48*01F | 96.77% (270/279nt) | Musmus IGKJ5*01F | --- | CQQSNS |
| | | | | | WPLTF |

**Table 7: Group IV, DMB4F4, DMB10B7, DMB13D11**

| Sequence | V-gene and allele | V-region identity % (nt) | J-gene and allele | D-gene and allele | AA |
|---|---|---|---|---|---|
| | | | | | Junction |
| V_{H} | Musmus IGHV1-18*0 1F, or Musmus IGHV1-26*01F | 88.89% (256/288nt) | Musmus IGHJ2*01F | Musmus IGHD5-1*01P | CARGLTY |
| | | | | | FDQW |
| V_{L} | Musmus IGKV4-68*01F | 97.46% (269/276nt) | Musmus IGKJ2*01F | --- | CQQWNSK |
| | | | | | PPITF |

**Table 8: DMC209**

| Sequence | V-gene and allele | V-region identity % (nt) | J-gene and allele | D-gene and allele | AA |
|---|---|---|---|---|---|
| | | | | | Junction |
| V_{H} | | | | | |
| V_{L} | | | | | |

By the term "***variant***" it is meant sequences of amino acids or nucleotides different from the sequences specifically identified herein, in which one or more amino acid residues or nucleotides are deleted, substituted, or added.

It should be appreciated that by the term "***added*** ", as used herein it is meant any addition of amino acid residues to the sequences described herein.

Variants encompass various amino acid substitutions. An amino acid "***substitution***" is the result of replacing one amino acid with another amino acid which has similar or different structural and/or chemical properties. Amino acid substitutions may be made based on similarity in polarity, charge, solubility, hydrophobicity, hydrophilicity, and/or the amphipathic nature of the residues involved.

Typically, variants encompass conservative amino acid substitutions. Conservative substitution tables providing functionally similar amino acids are well known in the art. For example, nonpolar (hydrophobic) amino acids include alanine, leucine, isoleucine, valine, proline, phenylalanine, tryptophan, and methionine; polar neutral amino acids include glycine, serine, threonine, cysteine, tyrosine, asparagine, and glutamine; positively charged (basic) amino acids include arginine, lysine, and histidine; and negatively charged (acidic) amino acids include aspartic acid and glutamic acid.

Each of the following eight groups contains other exemplary amino acids that are conservative substitutions for one another:
1) Alanine (A), Glycine (G);
2) Aspartic acid (D), Glutamic acid (E);
3) Asparagine (N), Glutamine (Q);
4) Arginine (R), Lysine (K);
5) Isoleucine (I), Leucine (L), Methionine (M), Valine (V);
6) Phenylalanine (F), Tyrosine (Y), Tryptophan (W);
7) Serine (S), Threonine (T); and
8) Cysteine (C), Methionine (M).

Conservative nucleic acid substitutions are nucleic acid substitutions resulting in conservative amino acid substitutions as defined above.

Variants also encompass non-polar to polar amino acid substitutions and *vice-versa.*

As used herein, the term "***amino acid***" or "***amino acid residue***" refers to naturally occurring and synthetic amino acids, as well as amino acid analogs and amino acid mimetics that function similarly to the naturally occurring amino acids.

Variant sequences refer to amino acids or nucleic acids sequences that may be characterized by the percentage of the identity of their amino acid or nucleotide sequences with the amino acid or nucleotide sequences described herein (for example, the amino acid or nucleotide sequences of the heavy and light chains of the antibodies herein described).

In some embodiments, variant sequences as herein defined refer to nucleic acid sequences that encode the heavy and light chain variable regions, each having a sequence of nucleotides with at least 70% or 75% of sequence identity, around 80% or 85% of sequence identity, around 90%, 91%, 92%, 93%, 94%, 95%, 96%, 97%, 98% or 99% of sequence identity when compared to the sequences of the heavy and light chain variable regions described herein.

In some other embodiments, variant sequences as herein defined refer to amino acid sequences of the heavy and light chain variable regions, each having a sequence of amino acids with at least 70% or 75% of sequence identity, around 80% or 85% of sequence identity, around 90%, 91%, 92%, 93%, 94%, 95%, 96%, 97%, 98% or 99% of sequence identity when compared to the sequences of the heavy and light chain variable regions described herein.

By the term "***activity of the antibodies***" it is meant the ability of the antibodies to bind MUC1 SEA domain, and preferably to mediate cell cytotoxicity either alone or as part of an immunocomplex with a cytotoxic moiety. The activity of the antibodies can be measured *in vivo* or *in vitro* using methods well known in the art, e.g., as described in the Examples below.

The binding of the antibody of the invention to its target protein may be measured for example using ELISA, biolayer interferometry (BLI), Western blot or immunofluorescence assays (IFA).

The biological activity of the antibodies can be measured for example in an *in vivo* cancer model, for example as detailed in the Examples below.

In another one of its aspects the present invention provides an isolated nucleic acid molecule comprising a nucleotide sequence encoding an antibody or antigen-binding fragment thereof according to the invention.

The term "***nucleic acid***" or "***nucleic acid molecule***" as herein defined refers to a polymer of nucleotides, which may be either single- or double-stranded, which is a polynucleotide such as deoxyribonucleic acid (DNA), and, where appropriate, ribonucleic acid (RNA). The terms should also be understood to include, as equivalents, analogs of either RNA or DNA made from nucleotide analogs, and, as applicable to the embodiment being described, single-stranded (such as sense or antisense) and double-stranded polynucleotides. The term DNA used herein also encompasses cDNA, i.e., complementary or copy DNA produced from an RNA template by the action of reverse transcriptase (RNA-dependent DNA polymerase).

The invention further provides an expression vector comprising the isolated nucleic acid molecule as herein defined.

"***Expression vector***" sometimes referred to as "***expression vehicle***" or "***expression construct***", as used herein, encompasses vectors such as plasmids, viruses, bacteriophage, integratable DNA fragments, and other vehicles, which enable the integration of DNA fragments into the genome of the host. Expression vectors are typically self-replicating DNA or RNA constructs containing the desired gene or its fragments, and operably linked genetic control elements that are recognized in a suitable host cell and effect expression of the desired genes. These control elements are capable of effecting expression within a suitable host. The expression vector in accordance with the invention may be competent with expression in bacterial, yeast, or mammalian host cells, to name but few.

In yet another one of its aspects the present invention provides a host cell transfected with the isolated nucleic acid molecule according to the invention or with the expression vector according to the invention.

The term "***host cells***" as used herein refers to cells which are susceptible to the introduction of the isolated nucleic acid molecule according to the invention or with the expression vector according to the invention. Preferably, said cells are mammalian cells, for example CHO cells or NS0 cells. Transfection of the isolated nucleic acid molecule or the expression vector according to the invention to the host cell may be performed by any method known in the art.

In yet another one of its aspects the present invention provides an immunoconjugate comprising the antibody or antigen-binding fragment thereof according to the invention and an additional cytotoxic or therapeutic agent as defined herein below.

The terms "***immunoconjugate***", *"**immunocomplex**"* are used interchangeably herein and refer to an antibody or antigen-binding fragment thereof according to the invention that is conjugated (linked or joined) to an additional agent. Immunoconjugates may be prepared by any method known to a person skilled in the art, for example, by cross-linking the additional agent to the antibody according to the invention or by recombinant DNA methods.

In certain embodiments, the immunoconjugate is an immunotoxin whereby the antibody or antigen-binding fragment thereof according to the invention is conjugated to a cytotoxic agent. The term "***cytotoxic agent***" as used herein refers to any agent that exerts a cytotoxic effect on a cell upon contact. Such cytotoxic agents are well known to a person skilled in the art. Examples of cytotoxic agents that may be used in the immunocomplex of the invention include, but are not limited to alkylating drugs, anthracyclines, pyrimidine derivatives, vinca alkaloids, photodynamic drugs, platinum-containing compounds, taxanes, topoisomerase inhibitors, ribosome inactivating agents (e.g., gelonin), agents that induce DNA damage (e.g., calicheamicin), tubulin inhibitors (e.g., emtansine), anti-mitotic agents (e.g., monomethyl auristatin), or bacterial toxins. The cytotoxic agents may also be radioisotopes or cytotoxic antibodies. In one embodiment, the toxic agent is Pseudomonas exotoxin, e.g., ZZ-PE38 (ZZ IgG-binding protein fused to Pseudomonas exotoxin).

The present invention also encompasses bispecific antibodies capable of binding to two separate targets or epitopes, wherein said bispecific antibody comprises the antibody or antigen-binding fragment thereof according to the invention and an additional antibody or antigen-binding fragment thereof.

Therefore, in a specific embodiment, the present invention provides an immunocomplex comprising an isolated monoclonal antibody or antigen-binding fragment thereof which binds to MUC1 SEA domain, wherein said antibody comprises:
a heavy chain complementarity determining region (CDRH) 1 denoted by SEQ ID NO. 25, CDRH2 denoted by SEQ ID NO. 26, CDRH3 denoted by SEQ ID NO. 27, and a light chain complementarity determining region (CDRL) 1 denoted by SEQ ID NO. 28, a CDRL2 denoted by SEQ ID NO. 29, and a CDRL3 denoted by SEQ ID NO. 30, and a cytotoxic agent being pseudomonas exotoxin, and wherein said immunocomplex reduces tumor volume upon administration to a subject with cancer.

The anti MUC1 SEA domain antibody of the invention may be administered in combination with at least one additional therapeutic agent.

The term "***additional therapeutic agent***" used herein refers to any agent that may be used for treating a disease or disorder, e.g., cancer.

In certain embodiments the additional therapeutic agent is an additional antibody. The term "***additional antibody***" as herein defined refers to antibodies of the invention (namely to the combined use of at least two antibodies of the invention) as well as to an antibody, which is not the antibody according to the invention, which may be used in combination with the antibody of the invention for treating a disease or disorder, e.g., cancer. Such other antibodies include but are not limited to anti-CD22 antibodies, anti-CD30 antibodies, anti-HER2 receptor antibodies, anti-VEGF antibodies, anti-EGFR antibodies, anti-tumor associated antigen (TAA) antibodies, and anti-check point inhibitors.

The additional therapeutic agent may also be a chemotherapeutic agent, or an antiinflammatory agent.

The present invention further provides a pharmaceutical composition comprising as an active ingredient at least one isolated anti-MUC1 SEA antibody of the invention, or antigen-binding fragment thereof or the immunoconjugate as herein defined and a pharmaceutically acceptable carrier, excipient, or diluent.

In a specific embodiment, said pharmaceutical composition is for use in the treatment of a disease or diorder associated with over-expression of MUC1.

The term ***"a disease or diorder associated with over-expression of MUC1"*** is used herein at its broadest sense and refers to any disease which is characterized by aberrant expression of MUC1. In a specific embodiment, the disease or disorder associated with over-expression of MUC1 is cancer. Examples include, but are not limited to, lung carcinoma, prostate carcinoma, breast carcinoma, ovarian carcinoma, colon carcinoma, small intestinal carcinoma, pancreatic carcinoma, gastric carcinoma, liver cancer, multiple myeloma, or acute myelogenous leukemia.

In other embodiments, the disease or disorder associated with over-expression of MUC1 is an autoimmune or inflammatory disease. Non-limiting examples of an autoimmune or inflammatory disease include rheumatoid arthritis, psoriatic arthritis, systemic lupus erythematosus, amyloidosis, and autoimmune pancreatitis.

In other embodiments, said disease or disorder is a non-malignant abnormal growth condition, e.g., cysts, for example clinically significant renal cysts, large, non-functional thyroid cysts and thyroid masses, hepatic cysts and the like.

The "***pharmaceutical composition***" of the invention generally comprises the antibody or any antigen-binding fragment thereof as herein defined and a buffering agent, an agent which adjusts the osmolarity of the composition and optionally, one or more pharmaceutically acceptable carriers, excipients and/or diluents as known in the art.

As used herein the term "***pharmaceutically acceptable carrier, excipient or diluent***" includes any solvent, dispersion medium, coating, antibacterial and antifungal agent, and the like, as known in the art. The carrier can be a solvent or dispersion medium containing, for example, water, ethanol, polyol (for example, glycerol, propylene glycol, and liquid polyethylene glycol, and the like), suitable mixtures thereof, and vegetable oils. Each carrier should be both pharmaceutically and physiologically acceptable in the sense of being compatible with the other ingredients and not injurious to the subject. Except as any conventional media or agent is incompatible with the active ingredient, its use in the therapeutic composition is contemplated.

In other embodiments the pharmaceutical composition according to the invention further comprises an additional therapeutic agent. Non-limiting examples of additional therapeutic agents include anti MUC1 antibodies, anti-CD22 antibodies, anti-CD30 antibodies, anti-HER2 receptor antibodies, anti-VEGF antibodies, anti-EGFR antibodies, anti-TAA antibodies and checkpoint inhibitors.

The present invention also provides methods of treatment or amelioration of a disease or disorder associated with over-expression of MUC1 (e.g. cancer) comprising administering to a subject in need thereof a therapeutically effective amount of the isolated monoclonal antibody or antigen-binding fragment thereof of the invention, or an immunoconjugate comprising the antibody or antigen-binding fragment thereof of the invention or a pharmaceutical composition comprising the isolated monoclonal antibody or antigen-binding fragment thereof or the immunoconjugate of the invention.

The terms ***"subject"*** or ***"patient"*** are used interchangeably and refer to a subject that may benefit from the present invention such as a mammal (e.g., canine, feline, ovine, porcine, equine, bovine, or human). In one specific embodiment the patient is human. Diagnosis of a disease or disorder associated with over-expression of MUC1 may be performed by a skilled physician by methods known in the art.

The term "***subject in need thereof***" in the context of the present invention *inter alia* refers to mammals, particularly human subjects suffering from a disease or disorder associated with over-expression of MUC1, as defined herein.

It is to be understood that the terms "***treat***", "***treating***", "***treatment***" or forms thereof, as used herein, mean reducing, preventing, curing, reversing, ameliorating, attenuating, alleviating, minimizing, suppressing, or halting the deleterious effects of a disease or a condition or delaying the onset of one or more clinical indications of a disease or disorder associated with over-expression of MUC1 (e.g., cancer), as defined herein. In some embodiments the methods according to the invention are wherein said methods further comprise administering to a subject in need thereof an additional therapeutic agent as herein defined.

Administration according to the present invention may be performed by any of the following routes: oral administration, intravenous, intramuscular, intraperitoneal, intrathecal, or subcutaneous injection; intra-rectal administration; intranasal administration, ocular administration, or topical administration.

In specific embodiments administration according to the present invention is performed intravenously.

The antibodies or antibody fragments as herein defined, any pharmaceutical compositions comprising the same or any conjugates comprising them may be administered to a subject in a single dose or in multiple doses.

A "***therapeutically effective amount***" of the isolated monoclonal antibody or any antigen-binding fragment thereof according to the invention, or the pharmaceutical composition according to the invention for purposes herein defined is determined by such considerations as are known in the art to cure, arrest or at least alleviate or ameliorate the medical condition. For any preparation used in the methods of the invention, the dosage or the therapeutically effective amount can be estimated initially from *in vitro* cell culture assays or based on suitable animal models.

In some embodiments the therapeutically effective amount in accordance with the invention is in the range of 10 µg/kg to about 50 mg/kg.

In other embodiments the therapeutically effective amount in accordance with the invention is in the range of 0.1 mg/kg to 40 mg/kg, 1 mg/kg to 10 mg/kg, or 5 mg/kg to 10 mg/kg.

Specific exemplary doses include, but are not limited to 0.25 mg/kg, or 0.75 mg/kg, or 2.5 mg/kg, or 5 mg/kg, or 10 mg/kg given as a daily dose, or once every three days, or once a week according to the physician's discretion. In one embodiment, the doses are given intravenously.

The present invention further provides the isolated anti MUC1 SEA antibody or any antigen-binding fragment thereof according to the invention, or the immunocomplex according to the invention, or the pharmaceutical composition according to the invention for use in a method of treatment or amelioration of a disease or disorder associated with over-expression of MUC1 (e.g., cancer) as defined herein.

Still further the present invention provides use of the isolated monoclonal antibody or antigen-binding fragment thereof, the immunocomplex, or the pharmaceutical composition of the invention in the preparation of a medicament for the treatment or amelioration of a disease or disorder associated with over-expression of MUC1 (e.g., cancer), as defined herein.

It is appreciated that the term "***purified***" or "***isolated***" refers to molecules, such as amino acid or nucleic acid sequences, peptides, polypeptides, or antibodies that are removed from their natural environment, isolated, or separated. An "isolated antibody" is therefore a purified antibody. As used herein, the term "***purified***" or "***to purify***" also refers to the removal of contaminants from a sample.

In another aspect, the present invention provides a method of diagnosing a disease or disorder (e.g., cancer) in a biopsy obtained from a subject, said method comprising:
a. contacting said biopsy with at least one isolated monoclonal antibody or antigen-binding fragment thereof of the invention; and
b. detecting said isolated monoclonal antibody or any antigen-binding fragment thereof;
wherein detection of cells over-expressing MUC1 SEA in the biopsy serves as an indication of said disease or disorder (e.g., cancer).

Assessment of the ability of the isolated antibodies of the present invention to detect MUC1-SEA expression may be performed by any method known in the art, for example by immunohistochemistry or flow cytometry. Immunohistochemistry may be performed on formaldehyde fixed sections from Fresh Frozen (FF) tissues as well as on Paraffin Embedded and Formaldehyde Fixed (PEFF) tissues. For example, as shown in Example 2 below, antibody DMB5F3 stained MUC1-expressing cells present in both FF and PEFF sections. The antibody was also capable of recognizing MUC1-expressing cells using flow cytometry. In various embodiments the isolated antibodies in accordance with the present invention may be labeled according to any methods known in the art. In other embodiments detection may be based on identifying said antibodies using secondary antibodies.

The term "***biopsy***" is used herein in its broadest sense and refers to any biopsy taken from a subject as herein defined in which cells overexpressing MUC1 SEA may be detected. Biopsies may be obtained from mammals (including humans) and encompass both fluid samples comprising cells, and tissue samples. In some embodiments the fluid sample is blood, plasma, serum, lymph fluid or urine. In some embodiments the biopsy is a tissue sample suspected of containing cancerous cells.

In another aspect, the present invention provides a method of imaging a disease or disorder, said method comprising:
a. introducing at least one isolated anti-MUC1 SEA monoclonal antibody or antigen-binding fragment thereof of the invention into a subject, wherein said antibody or antigen-binding fragment thereof are detectably labelled with a radioisotope, or with a visualizable agent (i.e., an agent that can be visualized, for example by scanning); and
b. visualizing said detectably labelled isolated anti-MUC1 SEA monoclonal antibody or any antigen-binding fragment thereof;
wherein detection of cells and/or tissues labelled with said isotope or with said visualizable agent indicates the presence, and/or the localization and/or the extent and/or the presence of metastases of said disease or disorder in said subject.

The term "***about***" as used herein indicates values that may deviate up to 1%, more specifically 5%, more specifically 10%, more specifically 15%, and in some cases up to 20% higher or lower than the value referred to, the deviation range including integer values, and, if applicable, non-integer values as well, constituting a continuous range. Disclosed and described, it is to be understood that this invention is not limited to the specific examples, methods steps, and compositions disclosed herein as such methods steps and compositions may vary somewhat. It is also to be understood that the terminology used herein is used for the purpose of describing specific embodiments only and not intended to be limiting since the scope of the present invention will be limited only by the appended claims and equivalents thereof.

It must be noted that, as used in this specification and the appended claims, the singular forms "a", "an" and "the" include plural referents unless the content clearly dictates otherwise.

Throughout this specification and the Examples and claims which follow, unless the context requires otherwise, the word "***comprise***", and variations such as "***comprises***" and "***comprising***", will be understood to imply the inclusion of a stated integer or step or group of integers or steps but not the exclusion of any other integer or step or group of integers or steps.

### EXAMPLES

### Materials and Methods

### Reagents and antibodies

All reagents and chemicals were obtained from Sigma (St. Louis, MO), unless otherwise specified. Secondary antibodies used in cell counter-staining or in immunohistochemical development were obtained from Jackson ImmunoResearch Laboratories (Bar Harbor, ME).

### Immunization of mice and generation of hybridomas

Mice were initially immunized with 5 consecutive intradermal DNA immunizations spaced at 21-day intervals. The immunizing DNA consisted of the pCL-MUC1-TM expression vector plasmid that codes for the MUC1-TM protein. The extracellular domain of MUC1-X protein (recombinant bacterial MUC1-Xex) together with incomplete Freund's adjuvant was then used to boost the mice. Bacterially synthesized recombinant MUC1-Xex protein used for these immunizations spontaneously self-cleaves, generating the MUC1-X a and b subunits that strongly, yet noncovalently, interact with each other forming the very stable heterodimeric cleaved MUC1-Xex protein. Hybridomas were prepared by fusion of non-secreting myeloma cells with immune splenocytes and screened by ELISA assay.

### ELISA for determining binding of anti-MUC1 polyclonal and monoclonal antibodies to the extracellular domain of the MUC1-X protein

Elisa Immunoassay plates (CoStar) were coated with recombinant MUC1 proteins followed by blocking. Spent culture media from the initial hybridomas was then applied to the wells. Following incubation, samples were removed, and the wells were washed with PBS/Tween. Detection of bound antibodies was done with horseradish peroxidase (HRP)-conjugated anti-mouse antibody.

### Two-tiered screening for selection of anti-MUC1 monoclonal antibodies

The primary screen of the hybridomas was carried out by assessing antibody binding to the extracellular domain of MUC1-X (MUC1-Xex) as described in the ELISA assay above. To select hybridomas secreting antibody that recognize not only MUC1-Xex but also the complete cell surface MUC1-TM protein, those hybridomas presenting a positive signal in the first screen were subjected to a second-tier screen. This consisted of flow cytometric analysis using mouse cell transfectants (termed DA3-TM cells) which express human MUC1-TM and, in parallel, the same parental cells (termed DA3-PAR cells) that do not express human MUC1. This procedure ensured selection of antibody that not only bound MUC1 moieties common to both the MUC1-X and MUC1-TM but also recognized the cell surface human MUC1-TM molecule as it is expressed by mammalian cells.

### Cell lines and cell cultures

DA3-PAR parental mouse mammary cells (which do not express human MUC1), DA3-TM mouse mammary cells stably transfected with cDNA (and encoding for the full-length human MUC1-TM), cell lines T47D and ZR75 (human breast carcinomas), cell lines KB (human epidermoid carcinoma), Colo357 (human pancreatic carcinoma), N87 (human gastric carcinoma), and CHO-K1 (Chinese hamster ovary cells) were all grown in Dulbecco's Modified Eagle's Medium (DMEM), RPMI, and DMEM:F12 (1:1) culture media [12].

### Animals

Seven-weeks-old athymic (nude) and SCID mice (Harlan Laboratories, Madison, WI) were maintained until sacrifice in facilities approved by the Tel Aviv University (TAU) Institutional Ethics Committee for Accreditation of Laboratory Animal Care, in accordance with the regulations and standards of the Israel Ministry of Health.

### Flow cytometry analysis

Following trypsinization, MUC1-expressing tumor cells were washed and incubated with DMB5F3 (0.5 µg/ml), with or without MUC1-Xex competitor (100 µg/ml), for 1hr at 4°C. After washing with FACS buffer, fluorescein-labeled goat anti-mouse IgG was added for 45 min at 4°C. Bound IgG was detected by flow cytometry on a FACSCalibur^{™} (Becton Dickinson).

### Immunohistochemical (IHC) staining

Microarrays of normal and malignant pancreatic and breast tissue were purchased from US Biomax (Derwood, MD). Automated Immunological stains were obtained with the use of the Dako Autostainer Link 48 (Dako, Santa Clara, CA), according to the manufacturer's instructions. Antigenic retrieval was carried out with citrate buffer, for 30 min at room temperature. Endogenous peroxidase activity was blocked by the addition of Envision Flex Peroxidase Blocking Reagent (Dako) for 30 min, followed by incubation with DMB5F3 (5 µg/ml) for 2hrs. The immunohistochemical reaction was detected by the addition of polymer dextran coupled with peroxidase and secondary antibodies for 15 min (EnVision-Flex/HRP, Dako) and diaminobenzidine for 10 min (DakoCytomation). This was followed by counter-staining with hematoxylin for 10 min.

### Sequence determination of anti-MUC1-SEA module monoclonal antibodies

RNA was isolated from the DMB hybridoma series with TRIzol^{®} Reagent 1, according to a technical manual for the reagent (Ambion Inc., Foster City, CA). The RNA sequence was determined as follows: cDNA was generated by reverse transcription of total RNA with the use of universal or isotype-specific anti-sense primers, according to the technical manual for PrimeScriptTM 1st Strand cDNA Synthesis Kit (Takara Bio Inc., Mountain View, CA). Amplification of VH and VL antibody fragments was carried out according to the standard operating procedure (GenScript, NJ, USA) which involves rapid amplification of cDNA ends, followed by separate cloning into a standard cloning vector. Clones with inserts of the correct sizes were sequenced by colony PCR and at least five colonies with such inserts were sequenced for each fragment, with the consensus sequence derived by alignment of the different clones.

### Construction of chimeric chDMB5F3 for mammalian expression in Chinese hamster ovary cells

Human chimeric DMB5F3 (chDMB5F3) was generated from mouse DMB5F3. The mammalian vectors pMAZ-IgH and pMAZ-IgL were used as backbones for expression of cDNA coding for the VH and VL regions of DMB5F3 fused to human γ1 heavy and human κ light chains, respectively [Mazor et al., J Immunol Methods, 321 (2007) 41-59; Mazor et al., Cancer Lett, 257 (2007) 124-135]. The generated pMAZ IgH-chDMB5F3 and pMAZ IgL-chDMB5F3 vectors were used for transfection, and the resultant chimeric antibody chDMB5F3 was expressed in CHO cells. Stably transfected CHO cells secreted chDMB5F3, which was purified by protein-A affinity chromatography.

### Preparation of the chDMB5F3: ZZ-PE38 immunocomplex

The generation of the chDMB5F3:ZZ-PE38 immunocomplex was carried out as described in Pichinuk et al [11]. Briefly, chDMB5F3 was mixed with purified recombinant ZZ-PE38 protein in 20 mM Hepes buffer at a 2-fold molar excess of ZZ-PE38 and incubated for 2 hours at 4°C. Excess ZZ-PE38 and unconjugated chDMB5F3 antibody were removed by passage through a Sephadex G200 sizing column.

### In vitro cell viability assay

T47D, KB, A431 and N87 cancer cells (20,000 cells/well) were seeded in 96-well cell culture plates and grown at 37°C in 5% CO₂. After seeding, the chDMB5F3: ZZ-PE38 immunocomplex was applied directly to the cells at a concentration of 100ng/ml. Negative controls consisted of target cells reacted with ZZ-PE38 toxin alone, unconjugated to chDMB5F3 antibody, or with chDMB5F3 monoclonal antibody alone, devoid of ZZ-PE38 toxin. Cell viability was assessed according to alkaline phosphatase activity/well. The results were calculated as the average of 2 to 3 experiments, performed in triplicate.

### ELISA for determination of the binding of chDMB5F3: ZZ-PE38 immunocomplex to MUC1-Xex protein

To quantitate chDMB5F3 levels in mouse serum, ELISA immunoassay plates were coated with recombinant MUC1-Xex protein (see Fig. 1C showing the schematic structure), followed by blocking. At Days 1, 7, 15 and 28 after a single dose of 5 µg chDMB5F3: ZZ-PE38 immunocomplex, the mouse sera were applied to the ELISA wells at doubling dilutions and bound antibody was detected with horseradish peroxidase-conjugated goat anti-human Fc antibody. The results were calculated as the average of 2 to 3 experiments, performed in triplicate.

### In vivo cytotoxicity assay

Two quantitatively measurable human tumor xenograft models were established, one in 7-weeks-old female athymic nude mice and one in 7-weeks-old SCID mice, by using Colo357, a MUC1⁺ human pancreatic cancer cell line. A total 3×10⁶ Colo357 cells suspended in a small volume (100 µl) of Hepes buffer were injected subcutaneously into the right flank of the mice. In both the nude and the SCID mouse studies, the mice were divided into three groups (5 mice/group): Group 1 received 5 µg chDMB5F3: ZZ-PE38 (0.25mg/kg), Group 2 received 5 µg non-specific human Ig: ZZ-PE38 (0.25mg/kg) and Group 3 received an equivalent volume of Hepes buffer alone. In the athymic nude mice (7-weeks-old female mice), administration of anti-MUC1 immunotoxin, non-specific immunotoxin or Hepes in the three experimental groups was initiated 24 hours after the injection of Colo357 cells. The injection protocol consisted of a total six intravenous (iv) administrations on Days 1, 6, 9, 15, 22 and 29 in each experimental group (see black arrows along x-axis, Figure 6). In the SCID mice (7-weeks-old female mice), chDMB5F3: ZZ-PE38, non-specific human Ig: ZZ-PE38, and Hepes were administered similarly starting 24 hours after injection of pancreatic tumor cells in each of the three groups. The overall protocol of administrations consisted of eight iv injections on Days 1, 4, 8, 11, 16, 24, 31 and 38 in each of the three groups. Tumor growth was assessed serially in each of the experimental groups with a digital caliper. Tumor volume was calculated according to the formula 0.5 x L x W², where L is tumor length, and W- tumor width as described in Tomayko and Reynolds (Cancer Chemother Pharmacol, 24 (1989) 148-154). All the animal experiments were approved by the TAU's Institutional Review Board.

### Statistics

Statistical analysis of *in vivo* tumor growth was performed according to a simple paired 1-tailed t test. A p value of less than 0.05 was considered statistically significant.

### Results

### Example 1: Generation and sequencing of the DMB mAbs that bind the cell-bound MUC1 α-β junction and characterization of the DMBSF3 mAb

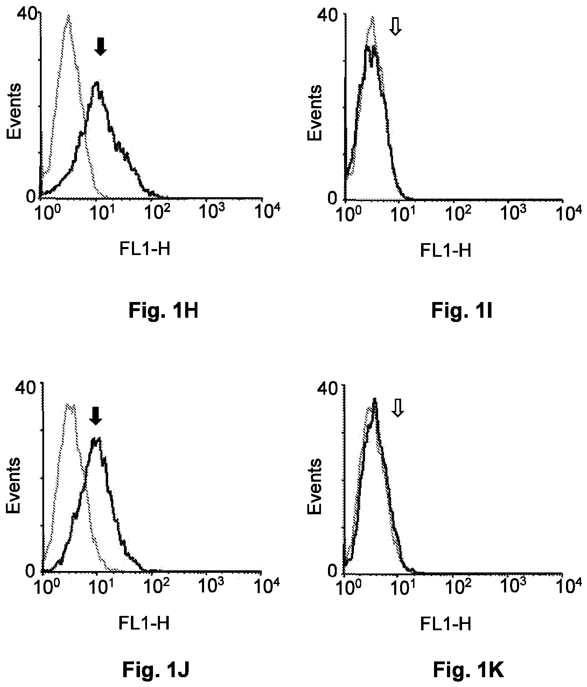

Anti-MUC1 monoclonal IgGs were generated from hybridomas produced with spleen cells isolated from inoculated mice having high titers of polyclonal anti-MUC1-Xex antibody. The MUC1-Xex recombinant protein used for immunization, and its relationship to the transmembrane MUC1-TM and MUC1-Xex proteins is shown in Fig. 1 (compare 1C with 1B and 1A). A total of seven DMB mAbs were thus generated.

The nucleotide sequences of the variable regions of all the anti-MUC1 SEA α-β junction monoclonal antibodies were determined as described above in Materials and Methods, and the deduced amino acid sequences of the mAbs are presented in Figure 2. Sequencing of the resultant mAbs showed that they clustered into 4 groups, denominated [I] DMB5F3^{[I]}, [II] DMB7F3^{[II]}, [III] DMB4B4^{[III-a]} and DMB10F10 ^{[III-b]} and [IV] DMB4F4^{[IV-a]}, DMB10B7^{[IV-b]} and DMB 13D11^{[IV-c]} (see Fig. 2 and Tables 1 and 2 for full nucleotide sequence and amino acid sequence). Sequences within each group revealed either unique mAb sequences as for DMB5F3^{[I]} and DMB7F3^{[II]} or mAbs with identical V_{H} and V_{L} sequences, as for Groups [III] and [IV].

The variable domains for all antibodies are typical of affinity maturation as expected from antibodies generated by prime-boost. All mAbs except for group [IV] mAbs DMB10B7^{[IV-b]} and DMB 13D11^{[IV-c]} were Ig-gamma1. Group [IV] contained three mAbs with identical V_{H} and V_{L} sequences- one (DMB4F4^{[IV-a]}) was of the Ig-gamma1 subtype, whereas the remaining two (DMB10B7^{[IV-b]} and DMB 13D11^{[IV-c]}), were IgA.

All seven anti-MUC1 α-β junction mAbs robustly bound to cells expressing the transmembrane MUC1-TM protein, as assessed by flow cytometry (Fig. 1D-1K). Representative mAbs from each of the four mAb groups were also assessed for their ability to detect MUC1-TM expression by immunohistochemistry performed on formaldehyde fixed sections from Fresh Frozen (FF) tissues as well as on Paraffin Embedded and Formaldehyde Fixed (PEFF) tissues. Antibody DMB5F3^{[I]} stained MUC1-expressing cells present in both FF and PEFF sections (see below), whereas the DMB7F3^{[II]} and mAbs from group [IV] stained only on FF sections- in contrast, group [III] mAbs while binding well to MUC1-expressing cells as assessed by flow cytometry, were non-reactive with both FF and PEFF sections (data not shown).

The VH domain is derived from mouse germline V gene IGHV3-1*02 with 9 somatic mutations. It is 105/122(86%) identical to the highest scoring identical sequence in an NCBI BlastP search. The VL domain (V-kappa) is derived from mouse germline V gene IGKV5-48*01 with 3 somatic mutations. It is 101/107(94%) identical to the highest scoring identical sequence in an NCBI BlastP search.

Flow cytometry analyses showed that DMB5F3 bound strongly to DA3 cells stably transfected with full length MUC1 (DA3-TM) (Fig. 1D), whereas non-transfected DA3-PAR cells, which do not express MUC1, were consistently negative (Fig. 1E). Colo357, a MUC1- positive human pancreatic cancer cell line, as well as the MUC1 positive breast cancer cell lines T47D and ZR75, exhibited strong reactivity with DMB5F3 (Fig. 1D, 1F, 1H and 1J). The addition of competing soluble recombinant MUC1-Xex protein (see Fig. 1C for the structure of recombinant MUC1-Xex), abolished all DMB5F3 cell binding (Fig. 1G, 1I and 1K), confirming antibody specificity.

### Example 2: IHC staining of human pancreatic and breast tissue sections with DMB5F3

To determine the degree to which monoclonal DMB5F3 binds malignant and normal tissues, a variety of malignancies in tissue microarrays, including breast, pancreatic, lung, prostate, and colon carcinomas underwent immunohistochemical staining (exemplary results are shown in Figures 3 and 4). The rationale to do extensive analyses for MUC1 expression, despite previous reports demonstrating MUC1 overexpression in malignancy lies in the significant fact that the anti-MUC1 mAbs described here recognize the MUC1 SEA module and were generated by immunization with recombinant MUC1-Xex protein (see Fig. 1C), and not with the MUC1-TM protein (see Fig. 1A). Analyses of MUC1 expression to date have almost exclusively been done with antibodies recognizing epitopes within the α-chain VNTR moiety. It was thus intriguing to analyze for the first time the cell recognition patterns of anti-MUC1-SEA domain antibodies. The tissue microarrays used for these analyses included the following (Biomax microarray designation in parenthesis): 6 pancreatic tumors with adjacent non-neoplastic tissues (PA241); 40 different pancreatic tumors and 8 normal pancreatic tissues (PA483); 3 samples each of breast plasma cell mastitis, adenosis and fibroadenoma and 36 invasive breast ductal carcinomas plus 2 invasive breast lobular carcinomas (BR963a) and 10 cases each of colon, breast, prostate, lung and colon carcinoma, in addition to two sections each from the corresponding normal tissues (TP481). A representative composite array of normal and malignant tissues immunohistochemically stained with DMB5F3 is shown in Fig. 3. Of the 46 pancreatic tumors (in microarrays PA241 and PA483), 44 exhibited strong reactivity with DMB5F3; tumor cells stained in a near-circumferential pattern (for examples see Fig. 3C and 3D). In contrast, normal pancreatic tissue DMB5F3 reactivity was restricted to the luminal surface of the ductal pancreatic epithelial cells (Fig. 3A).

Of the breast tissue samples analyzed on the BR963a microarray, minimal to no staining was seen in non-malignant tissues that included normal breast tissue, plasma cell mastitis, adenosis and fibroadenoma. In contrast, 21 of the 36 invasive breast ductal carcinomas showed very high DMB5F3 reactivity, 4 showed low levels of expression, and 11 samples showed little to no expression. The examined pancreatic tissues included both acinar (Fig. 3D) and ductal adenocarcinomas (Fig. 3C and Fig. 4N and 4O), and malignant breast tissues in the microarray consisted of invasive ductal carcinomas (Fig. 3G and 3H). Malignant cells from pancreatic carcinomas (Fig. 3C and 3D and Fig. 4N and 4O), breast carcinomas (Fig. 3G and 3H, and Fig. 3I-3N, patients 1-6) and lung, prostate and colon carcinomas (Fig. 4A, 4D and 4G, respectively, Figures 4B, 4E and 4H at higher magnifications) were strongly reactive with DMB5F3, with near-circumferential cellular staining. In contrast, normal pancreatic acinar cells showed only weak apical positivity (indicated by black arrows in Fig. 3A(i); higher magnification is shown in Fig. 3B, and Fig. 4M), consistent with a previous description [13]. Normal breast ductal epithelial cells (Fig. 3E and 3F) and normal gland-like structures formed by non-malignant epithelial cells adjacent to the malignancy on the microarray exhibited weak apical positivity (Fig. 3I-3N present biopsy sections from six patients; normal gland-like structures are indicated by black arrows). This was in marked contrast to malignant cells in the same section that stained strongly with DMB5F3 anti-MUC1-SEA antibody (Fig. 3I-3N). As malignant and non-malignant material were on the same microarray sample and, therefore simultaneously and uniformly stained, the possibility that mere technical differences in handling and staining could account for the findings may be excluded. Moreover, the fact that soluble MUC1-Xex competed-out cell staining by DMB5F3 confirmed the anti-MUC1 specificity of DMB5F3 (compare Fig. 3A and 3B, respectively).

To extend these observations to other tumor types, lung, prostate, and colon carcinomas were examined immunohistochemically with DMB5F3 (Fig. 4A, 4D and 4G). The results showed a similar pattern of MUC1 distribution to that observed in breast and pancreatic carcinomas (Fig 3). In addition to the increased density of MUC1 expression at the cellular level, distinctions in MUC1 architecture were also observed: Anti-MUC1-SEA DMB5F3 bound malignant cells in a near-circumferential pattern. Here too, staining with DMB5F3 was abolished in the presence of competing soluble MUC1-Xex protein, attesting to the specificity of DMB5F3 (data not shown) and no staining was observed with non-immune mouse immunoglobulin (compare Fig. 4A, 4D, 4G and 4J with 4C, 4F, 4I and 4L). The majority of the nearly 50 lung, prostate, colon, breast, and pancreatic adenocarcinoma tissues examined in the microarrays showed a similar IHC staining pattern, a minority expressing lower amounts of MUC1 and a few having negligible MUC1 expression. This non-uniformity is consistent with the heterogeneity of tumor phenotypes in general and of MUC1 particularly. Because pancreatic carcinoma, a high-mortality MUC1expressing malignancy was selected for *in vivo* studies (see below), pancreatic tumor tissue from an additional series of patients was examined to confirm the altered tumor-associated architecture of MUC1 expression (see representative staining in Fig. 4N and 4O). Although these analyses reveal increased circumferential DMB5F3 immunoreactivity over the entire cell surface of the adenocarcinoma cancer cells, the following two caveats are pertinent: (a) immunohistological analyses are only semiquantitative and (b) in some cases MUC1 is strongly expressed within the cell, rendering comparisons of surface expression difficult. Notwithstanding these two provisos, cancer cells derived from adenocarcinomas clearly exhibit high cell-surface immunoreactivity with DMB5F3.

### Example 3: In vitro cytotoxicity of the chDMB5F3: ZZ-PE38 immunocomplex

After demonstrating the reactivity of DMB5F3 with cancer cells expressing cell surface MUC1 both in cell lines (Fig 1D-1K) and in microarrays of tissue biopsies (Fig. 3 and 4), the antibody's ability to ferry a cytotoxic moiety into malignant cells was examined. The ZZ-PE38 fusion protein consists of Pseudomonas exotoxin PE38 and the IgG-binding ZZ-domain derived from Protein A. Because the ZZ domain binds tightly to human Fc and shows negligible binding to mouse IgG1 Fc, a chimeric DMB5F3 antibody was generated in which mouse IgG1-Fc was substituted for human Fc and then ZZ-PE38 added to the chimeric (ch)DM5F3 to form the immuno-toxin complex, as described in Materials and Methods.

As the ZZ-PE38 toxin alone is unable to bind to or internalize into cells, all tumor cytotoxicity induced by the DMB5F3-ZZ-P38- immunocomplex is due solely to cell binding and internalization by the anti-MUC1 DMB5F3 immunocomplex [Mazor et al., J Immunol Methods, 321 (2007) 41-59; Mazor et al., Cancer Lett, 257 (2007) 124-135]. The cell lines T47D, KB, A431 and N87 were cytometrically analyzed with chDMB5F3, Erbitux and Herceptin at concentrations of 300ng/ml. MUC1⁺ T47D cells (breast carcinoma) and KB cells (epidermoid tumors) were found to be sensitive to chDMB5F3: ZZ-PE38 immunocomplex-mediated inhibition of cell growth, with tumor cytotoxicity seen at antibody concentrations as low as 200pM (Fig. 5A and 5B. In contrast, T47D breast cancer cells expressing low, yet clearly detectable, levels of EGFR1 were insensitive to Erbitux^{®}: ZZ-PE38 (Fig. 5A, diamond shape tracing), and only partially sensitive to Herceptin^{®}: ZZ-PE38 (Fig. 5A, rectangle tracing). KB cells, which expressed low, yet clearly detectable levels of erbB2-EGFR2, were insensitive to Herceptin^{®}: ZZ-PE38 (Fig. 5B, rectangle tracing). Cells expressing markedly lower levels of MUC1 such as N87 showed approximately 40% cytotoxicity, contrasting with the high MUC1 expression and high cytotoxicity seen in T47D and KB cells (compare Fig. 5D, with Fig. 5A and 5B). A431, the lowest MUC1 expresser of all cell lines investigated, contained a major population of cells that showed no MUC1 expression with only a much smaller subpopulation expressing low-level MUC1. Consistent with this low level of MUC1 expression chDMB5F3: ZZ-PE38 immunocomplex resulted in a very limited cytotoxicity of A431. These results indicate that a threshold level of cell surface MUC1 expression and density is a necessary requirement to elicit cytotoxicity. A similar phenomenon is observed with the absence of cytotoxicity of Herceptin-immunotoxin-complex when applied to KB cells despite low, yet detectable, levels of erbB2-EGFR2 expression by these cells (Fig. 5B), and Erbitux-immunotoxin-complex when applied to T47D cells that also express low, yet detectable, levels of EGFR1 (Fig. 5A).

### Example 4: Pharmacokinetics of chDMB5F3 in nude mice

The *in vivo* stability of chDMB5F3 was evaluated by assessing serum levels on Days 1, 7, 14 and 28 following iv administration. The results showed that compared with Day 7, serum levels of antibody chDMB5F3 on Days 14 and 28 were twofold and fourfold decreased, respectively (Fig. 6D), consistent with previously reported half-lives in mice of *in vitro* generated chimeric IgGs and of antibodies in clinical use. As for the toxin conjugate, the half-life of Pseudomonas exotoxin has been shown to be extended by linkage to IgG.

### Example 5: In vivo cytotoxicity of chDMB5F3: ZZ-PE38 immunocomplex in xeno-transplanted human tumors

Administration of the chDMB5F3:ZZPE38 immunocomplex to nude mice xenotransplanted with MUC1⁺ human pancreatic Colo357 cells resulted in a marked cytocidal effect with a reduction in tumor volume at Days 21, 28 and 35 versus that in the control groups, which had received Hepes buffer or nonspecific, isotype-matched IgG-ZZ: PE38 (Fig. 6A). Upon completion of chDMB5F3:ZZPE38 immunocomplex administration, the tumor volume of the treated group, as expected, increased progressively in parallel with that in the control groups and by Day 40 (when the animals were sacrificed) the tumor volume in all three groups reached the 200-400 mm³ range (Fig. 6A). This again confirmed the tumor-suppressive effect of administered chDMB5F3:ZZPE38.

A factor conceivably limiting the cytotoxic efficacy of the chDMB5F3:ZZPE38 immunocomplex in xenotransplanted nude mice is endogenous circulating antibody, which by interacting with the ZZ linker may at least partially displace ZZ-PE38 toxin from chDMB5F3. Although ZZ does not bind mouse IgG1, it can bind mouse IgG2. Limitation in immunotoxin efficacy by displacement does not reflect defective binding of antibody chDMB5F3 to tumor cell surface MUC1 but arises from toxin loss owing to reduced ZZ-mediated linkage of ZZ-PE38 to the chDMB5F3 antibody. To avoid this complicating factor, a nearly identical study in SCID mice, which lack detectable endogenous antibody, was then performed. As in the nude mouse protocol, transplanted SCID mice were divided into three groups, one receiving the chDMB5F3:ZZPE38 immunocomplex, one receiving a matched-isotype IgG-ZZ:PE38, and one receiving Hepes buffer alone, each initiated 24 hours after injection of pancreatic tumor. As noted in the Materials Methods section, the protocol consisted of serial administrations in each group on Days 1, 4, 8, 11, 16, 24, 31, and 38. The chDMB5F3: ZZ-PE38 immunocomplex exhibited a marked antitumor effect: In SCID mice treated with chDMB5F3: ZZ-PE38 the volume of xenotransplanted Colo357 human tumors was reduced by as much as 90% versus that in the control groups (Figure 7). The actual values of post-treatment tumor volumes (in mm³) are as follows: Group 1 mice treated with the chDMB5F3-immunotoxin: 2, 14, 16, 25 and 36 mm³; Group 2 mice treated with the non-specific, isotype-matched IgG-ZZ:PE38: 180, 225, 258 and 270 mm³ (lack of tumor take for one mouse in this group is not included); and Group 3 mice treated with Hepes buffer alone: 180, 245, 304, 705 and 1008mm3. The extended scheduling of chDMB5F3: ZZ-PE38 administration to Days 31 and 38 assured an anti-tumor effect as late as Day 49.

### Example 6: Sequencing of DMC209

Total RNA was isolated from hybridoma cells following the technical manual of RNeasy Plus Micro Kit (QIAGEN, Cat. No.: 74034). Total RNA was then reverse-transcribed into cDNA using either isotype-specific anti-sense primers or universal primers following the technical manual of SMARTScribe Reverse Transcriptase (TaKaRa, Cat. No.: 639536). Antibody fragments of heavy chain and light chain were amplified according to the standard operating procedure (SOP) of rapid amplification of cDNA ends (RACE) of GenScript. Amplified antibody fragments were cloned into a standard cloning vector separately. Colony PCR was performed to screen for clones with inserts of correct sizes. The consensus sequence is provided in Table 1 (for the heavy chain variable region SEQ ID NO:11 and for the light chain variable region SEQ ID NO:12).

## Claims

1. An isolated monoclonal antibody or antigen-binding fragment thereof which binds to the MUC1 SEA domain, wherein said antibody comprises: a heavy chain complementarity determining region (CDRH) 1 denoted by SEQ ID NO. 25, CDRH2 denoted by SEQ ID NO. 26, CDRH3 denoted by SEQ ID NO. 27, and a light chain complementarity determining region (CDRL) 1 denoted by SEQ ID NO. 28, a CDRL2 denoted by SEQ ID NO. 29, and a CDRL3 denoted by SEQ ID NO. 30.

2. The isolated monoclonal antibody or antigen-binding fragment thereof according to claim 1, wherein said antibody or antigen-binding fragment thereof comprises a heavy chain variable region and a light chain variable region, wherein: said heavy chain variable region is encoded by a nucleic acid sequence which is at least 70% identical to the nucleic acid sequence denoted by SEQ ID NO. 1 and wherein said light chain variable region is encoded by a nucleic acid sequence which is at least 70% identical to SEQ ID NO. 2.

3. The isolated monoclonal antibody or antigen binding fragment according to claim 2 wherein said heavy chain variable region is encoded by a nucleic acid sequence which is at least 80% identical to the nucleic acid sequence denoted by SEQ ID NO. 1 and wherein said light chain variable region is encoded by a nucleic acid sequence which is at least 80% identical to SEQ ID NO. 2.

4. The isolated monoclonal antibody or antigen binding fragment according to claim 2 wherein said heavy chain variable region is encoded by a nucleic acid sequence which is at least 90% identical to the nucleic acid sequence denoted by SEQ ID NO. 1 and wherein said light chain variable region is encoded by a nucleic acid sequence which is at least 90% identical to SEQ ID NO. 2

5. The isolated monoclonal antibody according to any one of claims 1 to 4, wherein said antibody comprises: a heavy chain variable region comprising the amino acid sequence denoted by SEQ ID NO. 13 or a variant thereof and a light chain variable region comprising the amino acid sequence denoted by SEQ ID NO. 14, or a variant thereof.

6. The isolated monoclonal antibody or antigen-binding fragment thereof according to any one of claims 1 to 4, wherein said antibody is a mouse antibody, a chimeric antibody, a humanized antibody, or wherein said antigen-binding fragment thereof is Fv, single chain Fv (scFv), single chain Fv-Fc (scFv-Fc), Fab', Fab, F(ab')₂ or F(ab)₂.

7. An isolated nucleic acid molecule or expression vector comprising a nucleotide sequence encoding an antibody or any antigen-binding fragment thereof according to any one of claims 1-6.

8. An isolated host cell transfected with the expression vector according to claim 7.

9. An immunoconjugate comprising the antibody or antigen-binding fragment thereof according to any one of claims 1-6 and an additional cytotoxic or therapeutic agent.

10. The immunoconjugate according to claim 9 wherein said cytotoxic agent is selected from a group consisting of alkylating drugs, anthracyclines, pyrimidine derivatives, vinca alkaloids, photodynamic drugs, platinum-containing compounds, taxanes, topoisomerase inhibitors, ribosome inactivating agents, agents that induce DNA damage, tubulin inhibitors, anti-mitotic agents, radioisotopes, cytotoxic antibodies and bacterial toxins and pseudomonas exotoxin.

11. A bispecific antibody comprising the antibody or antigen-binding fragment thereof according to any one of claims 1-6 and a second antibody binding a different antigen target.

12. A pharmaceutical composition comprising as an active ingredient the isolated monoclonal antibody or antigen-binding fragment thereof according to any one of claims 1-6, or immunoconjugate according to any one of claims 9 or 10, the bispecific antibody of claim 11 and a pharmaceutically acceptable carrier, excipient, or diluent, optionally comprising an additional therapeutic agent.

13. A therapeutically effective amount of at least one isolated monoclonal antibody or antigen-binding fragment thereof according to any one of claims 1-6, or the immunoconjugate according to any one of claims 9 or 10, the bispecific antibody of claim 11, or the pharmaceutical composition according to claim 12 for use in the treatment or amelioration of a disease or disorder.

14. The therapeutically effective amount for use according to claim 13, wherein said disease or disorder is a MUC1 expressing cancer.

15. The therapeutically effective amount for use according to claim 14, wherein said cancer is selected from the group consisting of, lung carcinoma, prostate carcinoma, breast carcinoma, ovarian carcinoma, colon carcinoma, pancreatic carcinoma, multiple myeloma, and acute myelogenous leukemia.

16. A method of diagnosing a disease or disorder in a subject, wherein said disease or disorder is associated with MUC-1 expression, said method comprising:
a. contacting a biopsy obtained from said patient with at least one isolated monoclonal antibody or antigen-binding fragment thereof according to any one of claims 1-6; and
b. detecting said isolated monoclonal antibody or any antigen-binding fragment thereof;
wherein detection of cells over-expressing MUC1 SEA in the biopsy indicates that the subject is diagnosed with said disease or disorder.

17. An isolated anti-MUC1 SEA monoclonal antibody or antigen-binding fragment thereof according to any one of claims 1-6 for use as an imaging agent to image a disease or disorder associated with MUC1 expression in a subject in need thereof, wherein said antibody or antigen-binding fragment thereof are detectably labelled with a radioisotope or a visualizable agent in order to be visualized, and wherein the detection of cells and/or tissues labelled with said isotope or visualizable agent indicates the presence, the location and/or extent of metastases of said disease or disorder in said subject.

18. The isolated anti-MUC1 SEA monoclonal antibody or antigen-binding fragment thereof for use according to claim 17 wherein said disease or disorder is a MUC1 expressing cancer.

## Patentansprüche

1. Isolierter monoklonaler Antikörper oder Antigen-bindendes Fragment davon, das an die MUC1 SEA-Domäne bindet, wobei der Antikörper Folgendes aufweist: eine komplementaritätsbestimmende Region der schweren Kette (CDRH) 1, die mit SEQ ID NR. 25 bezeichnet ist, CDRH2, die mit SEQ ID NR. 26 bezeichnet ist, CDRH3, die durch SEQ ID NR. 27 bezeichnet ist, und eine komplementaritätsbestimmende Region der leichten Kette (CDRL) 1, die mit SEQ ID NR. 28 bezeichnet ist, eine CDRL2, die durch SEQ ID NR. 29 bezeichnet ist, und eine CDRL3, die durch SEQ ID NR. 30 bezeichnet ist.

2. Isolierter monoklonaler Antikörper oder Antigen-bindendes Fragment davon nach Anspruch 1, wobei der Antikörper oder das Antigen-bindende Fragment davon eine variable Region der schweren Kette und eine variable Region der leichten Kette aufweist, wobei: die variable Region der schweren Kette durch eine Nukleinsäuresequenz kodiert wird, die zu mindestens 70 % mit der Nukleinsäuresequenz, die durch SEQ ID NR. 1 bezeichnet ist, identisch ist, und wobei die variable Region der leichten Kette durch eine Nukleinsäuresequenz kodiert wird, die zu mindestens 70 % mit SEQ ID NR. 2 identisch ist.

3. Isolierter monoklonaler Antikörper oder Antigen-bindendes Fragment nach Anspruch 2, wobei die variable Region der schweren Kette durch eine Nukleinsäuresequenz kodiert wird, die zu mindestens 80 % mit der Nukleinsäuresequenz identisch ist, die mit SEQ ID NR. 1 bezeichnet ist, und wobei die variable Region der leichten Kette durch eine Nukleinsäuresequenz kodiert wird, die zu mindestens 80 % mit SEQ ID NR. 2 identisch ist.

4. Isolierter monoklonaler Antikörper oder Antigen-bindendes Fragment nach Anspruch 2, wobei die variable Region der schweren Kette durch eine Nukleinsäuresequenz kodiert wird, die zu mindestens 90 % mit der Nukleinsäuresequenz identisch ist, die mit SEQ ID NR. 1 bezeichnet ist, und wobei die variable Region der leichten Kette durch eine Nukleinsäuresequenz kodiert wird, die zu mindestens 90 % mit der SEQ ID NR. 2 identisch ist.

5. Isolierter monoklonaler Antikörper nach einem der Ansprüche 1 bis 4, wobei der Antikörper Folgendes aufweist: eine variable Region der schweren Kette, die die mit SEQ ID NR. 13 bezeichnete Aminosäuresequenz, oder eine Variante davon aufweist, und eine variable Region der leichten Kette, die die mit SEQ ID NR. 14 bezeichnete Aminosäuresequenz, oder eine Variante davon aufweist.

6. Isolierter monoklonaler Antikörper oder Antigen-bindendes Fragment davon nach einem der Ansprüche 1 bis 4, wobei der Antikörper ein Maus-Antikörper, ein chimärer Antikörper, ein humanisierter Antikörper ist, oder wobei das Antigen-bindende Fragment davon Fv, Einzelketten-Fv (scFv), Einzelketten-Fv-Fc (scFv-Fc), Fab', Fab, F(ab')2 oder F(ab)2 ist.

7. Isoliertes Nukleinsäuremolekül oder Expressionsvektor, das bzw. der eine Nukleotidsequenz aufweist, die einen Antikörper oder ein Antigen-bindendes Fragment davon nach einem der Ansprüche 1 bis 6 kodiert.

8. Isolierte Wirtszelle, die mit dem Expressionsvektor nach Anspruch 7 transfiziert ist.

9. Immunkonjugat, das den Antikörper oder ein Antigen-bindendes Fragment davon nach einem der Ansprüche 1 bis 6 und einen zusätzlichen zytotoxischen oder therapeutischen Wirkstoff aufweist.

10. Immunkonjugat nach Anspruch 9, wobei der zytotoxische Wirkstoff aus einer Gruppe ausgewählt ist, die sich aus alkylierenden Arzneimitteln, Anthrazyklinen, Pyrimidin-Derivaten, Vinca-Alkaloiden, photodynamischen Arzneimitteln, platinhaltigen Verbindungen, Taxanen, Topoisomerase-Inhibitoren, Ribosomeninaktivierenden Wirkstoffen, Wirkstoffen, die DNA-Schäden induzieren, Tubulin-Inhibitoren, anti-mitotischen Mitteln, Radioisotopen, zytotoxischen Antikörpern und bakteriellen Toxinen und Pseudomonas-Exotoxin zusammensetzt.

11. Bispezifischer Antikörper, der den Antikörper oder ein Antigen-bindendes Fragment davon nach einem der Ansprüche 1 bis 6 und einen zweiten Antikörper aufweist, der ein anderes Antigenziel bindet.

12. Pharmazeutische Zusammensetzung, die als Wirkstoff den isolierten monoklonalen Antikörper oder ein Antigen-bindendes Fragment davon nach einem der Ansprüche 1 bis 6 oder ein Immunkonjugat nach einem der Ansprüche 9 oder 10, den bispezifischen Antikörper nach Anspruch 11 und einen pharmazeutisch annehmbaren Träger, Hilfsstoff oder ein Verdünnungsmittel, optional ein zusätzliches therapeutisches Mittel aufweisend, aufweist.

13. Therapeutisch wirksame Menge mindestens eines isolierten monoklonalen Antikörpers oder Antigen-bindenden Fragments davon nach einem der Ansprüche 1 bis 6 oder des Immunkonjugats nach einem der Ansprüche 9 oder 10, des bispezifischen Antikörpers nach Anspruch 11 oder der pharmazeutischen Zusammensetzung nach Anspruch 12 zur Verwendung bei der Behandlung oder Linderung einer Krankheit oder Störung.

14. Therapeutisch wirksame Menge zur Verwendung nach Anspruch 13, wobei die Krankheit oder Störung ein Krebs ist, der MUC1 exprimiert.

15. Therapeutisch wirksame Menge zur Verwendung nach Anspruch 14, wobei der Krebs ausgewählt ist aus der Gruppe, die sich aus Lungenkarzinom, Prostatakarzinom, Brustkarzinom, Ovarialkarzinom, Kolonkarzinom, Pankreaskarzinom, multiplem Myelom und akuter myeloischer Leukämie zusammensetzt.

16. Verfahren zur Diagnose einer Krankheit oder Störung bei einem Patienten, wobei die Krankheit oder Störung mit MUC-1-Expression assoziiert ist, wobei das Verfahren Folgendes umfasst:
a. Inkontaktbringen einer dem Patienten entnommenen Biopsie mit mindestens einem isolierten monoklonalen Antikörper oder einem Antigen-bindenden Fragment davon nach einem der Ansprüche 1 bis 6; und
b. Nachweisen des isolierten monoklonalen Antikörpers oder eines Antigen-bindenden Fragments davon;
wobei der Nachweis von Zellen, die MUC1 SEA in der Biopsie überexprimieren, anzeigt, dass der Patient von der Krankheit oder Störung betroffen ist.

17. Isolierter monoklonaler Anti-MUCl-SEA-Antikörper oder Antigen-bindendes Fragment davon nach einem der Ansprüche 1 bis 6 zur Verwendung als Bildgebungsmittel zur Bildgebung bei einer Krankheit oder Störung, die mit der MUC1-Expression bei einem Patienten assoziiert ist, der dies benötigt, wobei der Antikörper oder das Antigen-bindende Fragment davon nachweisbar mit einem Radioisotop oder einem Wirkstoff, der sichtbar gemacht werden kann, markiert sind, um sichtbar gemacht zu werden, und wobei der Nachweis von Zellen und/oder Geweben, die mit dem Isotop oder dem Wirkstoff, der sichtbar gemacht werden kann, markiert sind, das Vorhandensein, die Stelle und/oder die Ausdehnung von Metastasen der Krankheit oder Störung beim Patienten anzeigt bzw. anzeigen.

18. Isolierter monoklonaler Anti-MUCl-SEA-Antikörper oder Antigen-bindendes Fragment davon zur Verwendung nach Anspruch 17, wobei die Krankheit oder Störung ein Krebs ist, der MUC1 exprimiert.

## Revendications

1. Anticorps monoclonal isolé ou fragment à liaison antigène de celui-ci qui se lie au domaine SEA de MUC1, dans lequel ledit anticorps comprend : une région déterminant la complémentarité de chaîne lourde (CDRH) 1 désignée par SEQ ID N° 25, une CDRH2 désignée par SEQ ID N° 26, une CDRH3 désignée par SEQ ID N° 27, et une région déterminant la complémentarité de chaîne légère (CDRL) 1 désignée par SEQ ID N° 28, une CDRL2 désignée par SEQ ID N° 29, et une CDRL3 désignée par SEQ ID N° 30.

2. Anticorps monoclonal isolé ou fragment à liaison antigène de celui-ci selon la revendication 1, dans lequel ledit anticorps ou fragment à liaison antigène de celui-ci comprend une région variable de chaîne lourde et une région variable de chaîne légère, dans lequel : ladite région variable de chaîne lourde est codée par une séquence d'acides nucléiques qui est au moins identique à 70 % à la séquence d'acides nucléiques désignée par SEQ ID N° 1 et dans lequel ladite région variable de chaîne légère est codée par une séquence d'acides nucléiques qui est au moins identique à 70 % à SEQ ID N° 2.

3. Anticorps monoclonal isolé ou fragment à liaison antigène selon la revendication 2, dans lequel ladite région variable de chaîne lourde est codée par une séquence d'acides nucléiques qui est au moins identique à 80 % à la séquence d'acides nucléiques désignée par SEQ ID N° 1 et dans lequel ladite région variable de chaîne légère est codée par une séquence d'acides nucléiques qui est au moins identique à 80 % à SEQ ID N° 2.

4. Anticorps monoclonal isolé ou fragment à liaison antigène selon la revendication 2, dans lequel ladite région variable de chaîne lourde est codée par une séquence d'acides nucléiques qui est au moins identique à 90 % à la séquence d'acides nucléiques désignée par SEQ ID N° 1 et dans lequel ladite région variable de chaîne légère est codée par une séquence d'acides nucléiques qui est au moins identique à 90 % à SEQ ID N° 2

5. Anticorps monoclonal isolé selon l'une quelconque des revendications 1 à 4, dans lequel ledit anticorps comprend : une région variable de chaîne lourde comprenant la séquence d'acides aminés désignée par SEQ ID N° 13 ou un variant de celle-ci et une région variable de chaîne légère comprenant la séquence d'acides aminés désignée par SEQ ID N° 14, ou un variant de celle-ci.

6. Anticorps monoclonal isolé ou fragment à liaison antigène de celui-ci selon l'une quelconque des revendications 1 à 4, dans lequel ledit anticorps est un anticorps de souris, un anticorps chimérique, un anticorps humanisé, ou dans lequel ledit fragment à liaison antigène de celui-ci est Fv, Fv simple chaîne (scFv), Fv simple chaîne-Fc (scFv-Fc), Fab', Fab, F(ab')2 ou F(ab)2.

7. Molécule d'acide nucléique isolée ou vecteur d'expression comprenant une séquence nucléotidique codant pour un anticorps ou un quelconque fragment à liaison antigène de celui-ci selon l'une quelconque des revendications 1 à 6.

8. Cellule hôte isolée transfectée avec le vecteur d'expression selon la revendication 7.

9. Immunoconjugué comprenant l'anticorps ou le fragment de liaison à l'antigène de celui-ci selon l'une quelconque des revendications 1 à 6 et un agent cytotoxique ou thérapeutique supplémentaire.

10. Immunoconjugué selon la revendication 9, dans lequel ledit agent cytotoxique est choisi dans le groupe constitué par les médicaments alkylants, les anthracyclines, les dérivés de pyrimidine, les alcaloïdes de la pervenche, les médicaments photodynamiques, les composés contenant du platine, les taxanes, les inhibiteurs de topoisomérase, les agents inactivant les ribosomes, les agents qui induisent une détérioration de l'ADN, les inhibiteurs de la tubuline, les agents antimitotiques, les radioisotopes, les anticorps cytotoxiques et les toxines bactériennes et exotoxine de pseudomonas.

11. Anticorps bispécifique comprenant l'anticorps ou un fragment à liaison antigène de celui-ci selon l'une quelconque des revendications 1 à 6 et un deuxième anticorps se liant à une cible antigénique différente.

12. Composition pharmaceutique comprenant comme ingrédient actif l'anticorps monoclonal isolé ou un fragment à liaison antigène de celui-ci selon l'une quelconque des revendications 1 à 6, ou un immunoconjugué selon l'une quelconque des revendications 9 ou 10, l'anticorps bispécifique selon la revendication 11 et un véhicule, excipient ou diluant pharmaceutiquement acceptable, comprenant facultativement un agent thérapeutique supplémentaire.

13. Quantité thérapeutiquement efficace d'au moins un anticorps monoclonal isolé ou fragment à liaison antigène de celui-ci selon l'une quelconque des revendications 1 à 6, ou de l'immunoconjugué selon l'une quelconque des revendications 9 ou 10, de l'anticorps bispécifique selon la revendication 11, ou de la composition pharmaceutique selon la revendication 12 pour une utilisation dans le traitement ou l'amélioration d'une maladie ou d'un trouble.

14. Quantité thérapeutiquement efficace pour une utilisation selon la revendication 13, dans laquelle ladite maladie ou ledit trouble est un cancer exprimant MUC1.

15. Quantité thérapeutiquement efficace pour une utilisation selon la revendication 14, dans laquelle ledit cancer est choisi dans le groupe constitué par le carcinome du poumon, le carcinome de la prostate, le carcinome du sein, le carcinome de l'ovaire, le carcinome du côlon, le carcinome pancréatique, le myélome multiple et la leucémie myélogène aiguë.

16. Procédé de diagnostic d'une maladie ou d'un trouble chez un sujet, dans lequel ladite maladie ou ledit trouble est associé à l'expression de MUC-1, ledit procédé comprenant :
a. la mise en contact d'une biopsie obtenue à partir dudit patient avec au moins un anticorps monoclonal isolé ou fragment à liaison antigène de celui-ci selon l'une quelconque des revendications 1 à 6 ; et
b. la détection dudit anticorps monoclonal isolé ou d'un quelconque fragment à liaison antigène de celui-ci ;
dans lequel la détection de cellules surexprimant SEA de MUC1 dans la biopsie indique que ladite maladie ou ledit trouble sont diagnostiqués chez le sujet.

17. Anticorps monoclonal anti-SEA MUC1 isolé ou fragment à liaison antigène de celui-ci selon l'une quelconque des revendications 1 à 6, pour une utilisation comme agent d'imagerie afin d'imager une maladie ou un trouble associés à l'expression de MUC1 chez un sujet en ayant besoin, dans lequel ledit anticorps ou ledit fragment à liaison antigène de celui-ci sont marqués de manière détectable avec un radio-isotope ou un agent visualisable afin d'être visualisés, et dans lequel la détection de cellules et/ou de tissus marqués avec ledit isotope ou ledit agent visualisable indique la présence, la localisation et/ou l'étendue de métastases de ladite maladie ou dudit trouble chez ledit sujet.

18. Anticorps monoclonal anti-SEA MUC1 isolé ou fragment de liaison à l'antigène de celui-ci pour une utilisation selon la revendication 17, dans lequel ladite maladie ou ledit trouble est un cancer exprimant MUC1.
